# EUROPEAN PATENT APPLICATION

(11) **EP 0 857 780 A1**
(43) Date of publication of application: **12.08.1998**
(21) Application number: 96930419.5
(22) Date of filing: 17.09.1996
(51) Int. Cl.: C12N 15/11, C12P 21/02, C12P 21/08, C12Q 1/68, G01N 33/53, G01N 33/574, G01N 33/577, C07K 14/82

(54) **NOVEL PROTEINS REMARKABLY EXPRESSED IN LIVER CANCER, GENES ENCODING THE SAME, ANTIBODIES AGAINST THE SAME, AND METHOD FOR DETECTING THE EXPRESSION OF THE SAME**

(30) Priority: 14.09.1995 JP 236264/95; 27.11.1995 JP 331023/95; 20.06.1996 JP 179885/96; 30.08.1996 JP 229469/96
(71) Applicant: SUMITOMO ELECTRIC INDUSTRIES, LTD, Osaka-shi, Osaka-fu 541 (JP)
(72) Inventor: KISHIMOTO, Toshihiko, Sumitomo Electric Ind. Ltd., Yokohama-shi, Kanagawa 244 (JP); TAMURA, Taka-aki, Chiba University, Chiba-shi, Chiba 263 (JP); MAKINO, Yasutaka, Chiba University, Chiba-shi, Chiba 263 (JP); KOKURA. Kenji, Chiba University, Chiba-shi, Chiba 263 (JP); KUMAGAI, Yoichi, Chiba University, Tokyo 134-0088 (JP)
(74) Representative: Kolb, Helga, Dr. Dipl.-Chem.
(86) International application number: JP9602654
(87) International publication number: WO9710333

(57) **Abstract**

Novel proteins (CRTI, HRPI and GADII) showing elevated expression in the course of carcinogenesis; novel genes encoding these proteins; antisense genes of these genes; antibodies against these proteins; and a method for monitoring liver cancer by using the above-mentioned proteins, genes or antibodies. Genes expressed specifically in rat liver cancer are extracted and those showing elevated expression in liver cancer are isolated. Their cDNAs of the full length are obtained and thus the base sequences of these genes are determined. Thus it is confirmed that these genes are those specific to liver cancer. Further, the amino acid sequences encoded by the above-mentioned genes are determined and proteins having these amino acid sequences are expressed in recombinant *Escherichia coli* . Furthermore, antibodies against these proteins are constructed. Then it is clarified that the expression of these proteins can be detected by using the above-mentioned antibodies and the onset of liver cancer can be monitored by detecting the above-mentioned genes.

## Description

### Technical Field

This invention relates to proteins expressed markedly in hepatic cancer, genes encoding the proteins, antibodies to the proteins, and methods for detecting the expressions of the proteins or the genes.

### Background Art

Carcinoma or cancer is known to occur because of some abnormality in a gene. A change or abnormality in the gene at its transcription level, in particular, is regarded as a major cause of cancer ("Science" Vo1. 222, 1983, pp. 765-771). To elucidate the mechanism of onset of carcinoma, the acquisition of a protein variedly expressed during carcinogenesis and a gene encoding the protein, or a protein different in the state of expression among tissues and a gene encoding the protein has been performed eagerly since about 1980. For example, cancer-specific proteins, α-fetoprotein and CEA, are known which were acquired by biochemically analyzing a carcinomatous tissue, and searching for its difference from a normal tissue.

Methods used to identify these substances comprised preparing monoclonal antibodies to a carcinomatous tissue, and sorting antibodies reactive only with the cancer tissue from the resulting monoclonal antibodies, and then identifying a substance which would serve as an antigen reactive with the monoclonal antibodies.

Information obtained by these methods was mostly concerned with a protein, and no gene was obtained directly. To acquire a gene from the protein obtained, therefore, the gene was selected from a population of genes, called a gene library, by a genetic engineering technique.

Concretely, this method of selection is described in "Molecu1ar C1oning Second Edition" (Co1d Spring Harbor Laboratory Press, 1989), chapters 8, 9 and 12. As shown there, the method prepares a gene probe on the basis of information on the resulting protein, and acquires a gene corresponding to this gene probe from the gene library by a method called colony/plaque hybridization.

The same book also shows in chapters 8, 9, 11 and 12 a method for screening the desired gene from the gene library by use of monoclonal antibodies.

With these methods, however, the desired protein should be large in amount, or the use of the monoclonal antibodies requires an antigen protein of the cell surface type and with high antigenicity. A protein which obviates these requirements has been very difficult to obtain. Consequently, it has also been extremely difficult to yield a gene encoding the protein.

The present invention uses methods entirely different from the foregoing some known methods, to isolate genes whose expression in a hepatic cancer tissue is increased compared with a normal hepatic tissue.

That is, an object of the present invention is to provide novel proteins increasing in expression during a carcinogenic process, novel genes encoding the proteins, and antibodies to the proteins.

It is another object of the invention to provide methods for detecting the expressions of the proteins or the genes to monitor the onset and progress of hepatic carcinoma.

### Disclosure of the Invention

To attain the above objects, the inventors conducted extensive studies. As a result, genes specifically expressed in rat hepatic cancer were extracted by the subtraction method, and genes whose expression was increased in hepatic cancer were isolated by dot screening. From a hepatic cancer cDNA library, the full length cDNA's of the genes, and the base sequences of the genes were determined. Further, amino acid sequences encoded by the genes were determined.

Moreover, Northern blot hybridization confirmed the cDNA's to be hepatic cancer specific genes. Also, proteins encoded by the cDNA's were expressed in recombinant Escherichia coli (E. coli), and the proteins were confirmed to have the same functions as those of their naturally occurring equivalents.

Novel rat proteins CRTI (or HTF), HRPI and GADII (or CSAD) specific for hepatic cancer, and genes encoding these proteins were isolated by the above-described means. A novel human protein, GADII, specific for hepatic cancer, and a gene encoding this protein were also isolated.

The above proteins were each inoculated into a mammal other than a species from which the protein was derived (excluding human), to produce antibodies against the protein and confirm the antigenicity of the protein.

It was also found that the above genes, CRTI gene, HRPI gene and GADII gene, were detected in tissues of the rat and the human by Northern blot hybridization.

These findings showed that the onset and progress of cancer, especially hepatic cancer, could be monitored by detecting the expression of the proteins by use of those antibodies, and that the onset and progress of cancer, especially hepatic cancer, could be monitored by detecting the genes encoding the proteins.

### Brief Description of the Drawings

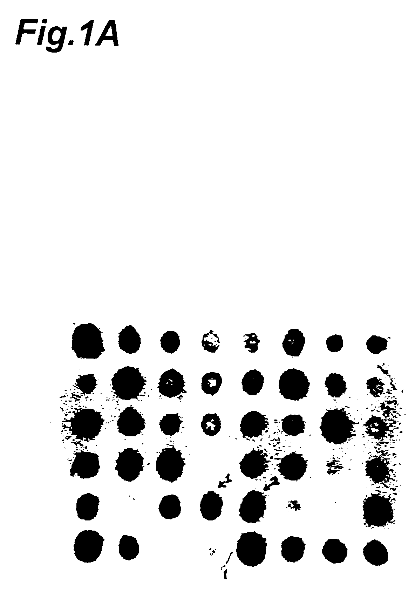

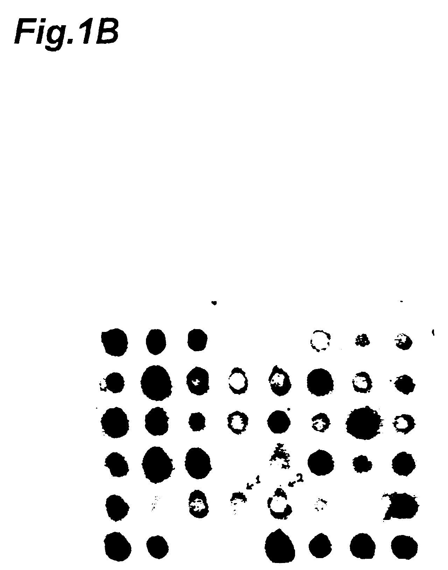

Figs. 1A and 1B are views showing the results of dot blot screening performed by fixing to a membrane DNA that was extracted from a gene library obtained from hepatic cancer by the subtraction method in Example 1. Fig. 1A is a view showing the results of screening with a cDNA probe prepared from hepatic cancer polyA RNA, while Fig. 1B is a view showing the results of screening with a cDNA probe prepared from normal liver polyA RNA. An arrow 1 indicates a dot of a gene whose expression is increased in hepatic cancer, and an arrow 2, a dot of a gene whose expression is increased in hepatic cancer.

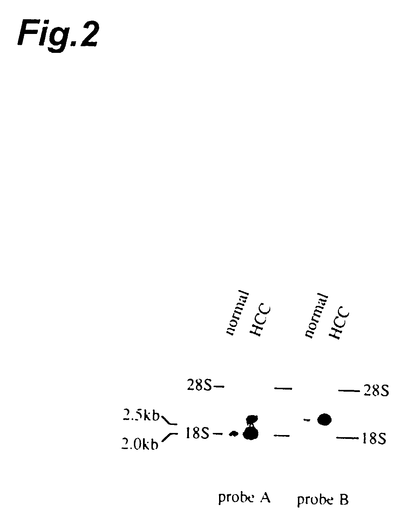

Fig. 2 is a photograph showing the results of analysis by Northern blot hybridization in systems of the rat normal liver and 7-month hepatic cancer by use of probes prepared in Examples 3 and 4 (probe A and probe B), respectively, with the left lane showing the normal liver (normal), and the right lane showing hepatic cancer (HCC).

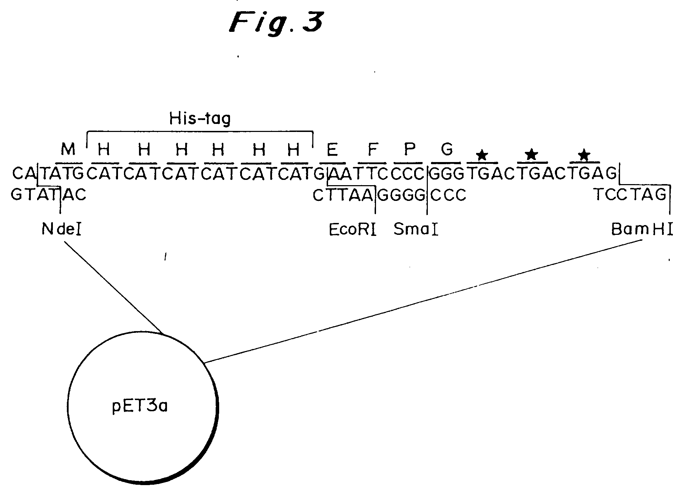

Fig. 3 is a view showing pET3a vector used in Examples 3, 14 and 16.

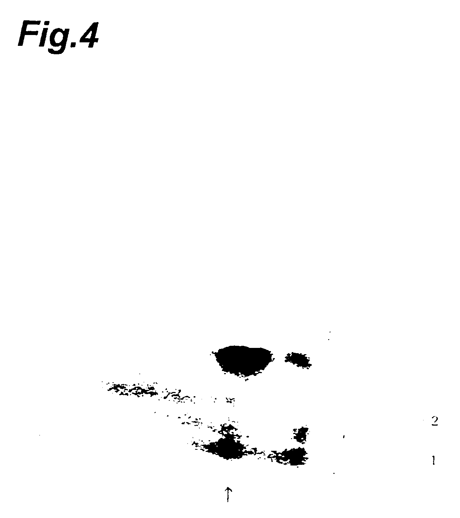

Fig. 4 is a photograph showing the results of analysis by Northern blot hybridization using gel electrophoresis of total RNA's prepared from a human hepatic cancer tissue and a non-cancerous hepatic tissue of the same patient. Lane 1 gives the results with the electrophoresed human hepatic cancer RNA, while lane 2 gives the results with the electrophoresed human non-cancerous RNA (a normal portion of the same patient). The band indicated by an arrow is the targeted band.

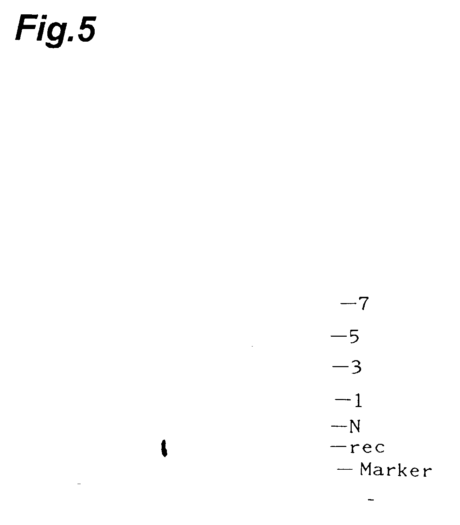

Fig. 5 is a photograph showing the results of Western blotting (protein level analysis) of various hepatic cancer tissues prepared in Example 1. From the bottom of the drawing upwards lie a marker (for a ladder), rec (positive control, CRTI prepared in Example 4), N (normal liver), 1 (liver one month after onset of cancer), 3 (liver 3 months after cancer onset), 5 (liver 5 months after cancer onset) and 7 (liver 7 months after cancer onset).

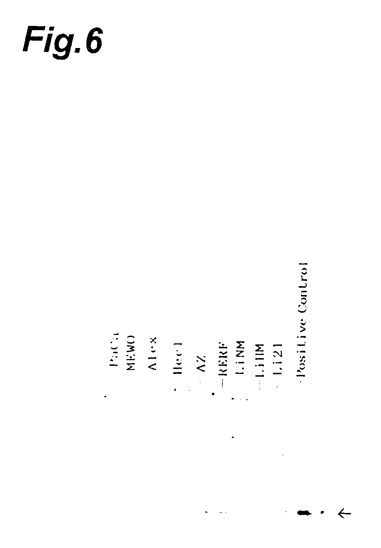

Fig. 6 is a photograph showing the results of Western blotting using various human cancer cells. From right to left in the drawing, lie a positive control, Li21, LiHM, LiNM (human hepatic cancer cells), RERF (human pulmonary cancer cells), AZ (human gastric cancer cells), Hecl (human uterine cancer cells), Alex (human hepatic cancer cells), MEWO (human melanoma), and PaCa (human pancreatic cancer cells). A band indicated by an arrow in the drawing represents CRTI.

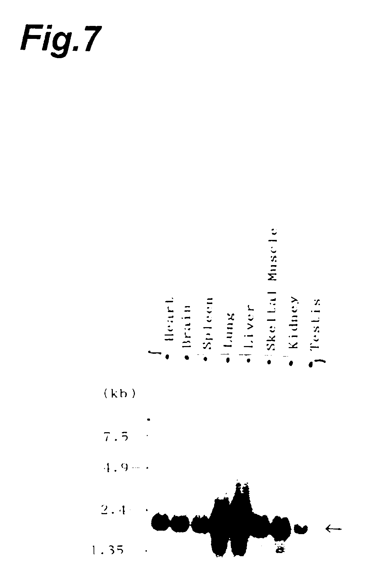

Fig. 7 is a photograph showing the results of Northern blot hybridization of CRTI gene distributed in various rat tissues. A band indicated by an arrow in the drawing is a band derived from CRTI gene.

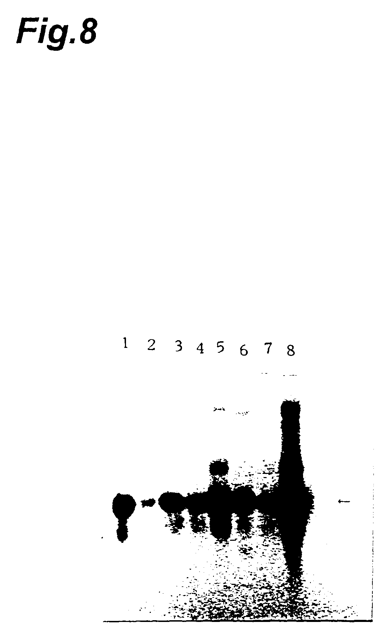

Fig. 8 is a photograph showing the results of Northern blot hybridization of CRTI gene distributed in various human tissues. A band indicated by an arrow in the drawing is a band derived from CRTI gene. Lane 1 represents heart, 2 brain, 3 placenta, 4 lung, 5 liver, 6 skeletal muscle, 7 kidney, and 8 pancreas.

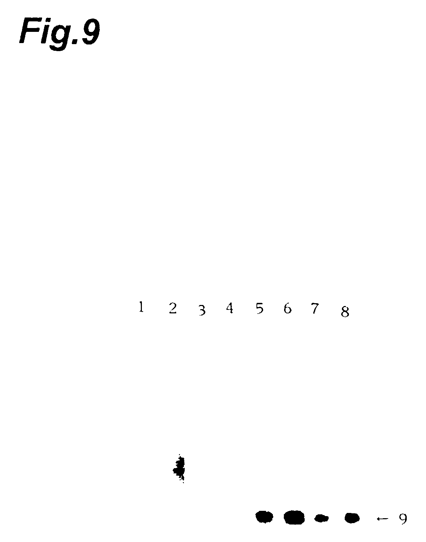

Fig. 9 is a view showing the results of analysis by Northern blot hybridization of mRNA's from normal liver and hepatic cancer with the use of HRPI gene as a probe. Lane 1 is a lane for normal liver mRNA, Lane 2 is a lane for mRNA of the hepatic cancer 12 hours after administration of DEN, Lane 3 is a lane for mRNA of the hepatic cancer 24 hours after DEN administration, Lane 4 is a lane for mRNA of the hepatic cancer 48 hours after DEN administration, Lane 5 is a lane for mRNA of the hepatic cancer 1 month after DEN administration, Lane 6 is a lane for mRNA of the hepatic cancer 3 months after DEN administration, Lane 7 is a lane for mRNA of the hepatic cancer 5 months after DEN administration, Lane 8 is a lane for mRNA of the hepatic cancer 7 months after DEN administration, and arrow 9 shows the position of the band of HRPI mRNA.

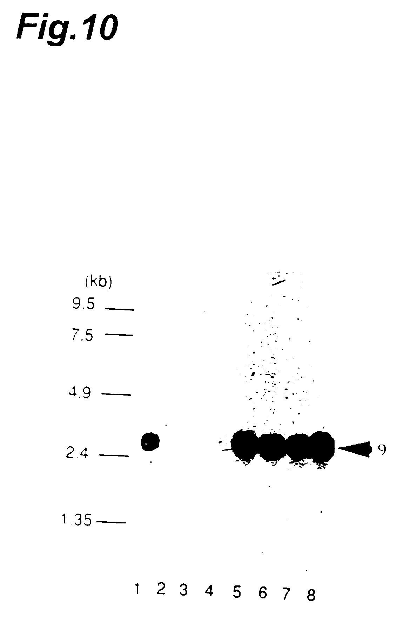

Fig. 10 is a view showing the results of analysis by Northern blot hybridization of mRNA's from normal liver and hepatic cancer with the use of GADII gene as a probe. Lane 1 is a lane for normal liver mRNA, Lane 2 is a lane for mRNA of the hepatic cancer 12 hours after administration of DEN, Lane 3 is a lane for mRNA of the hepatic cancer 24 hours after DEN administration, Lane 4 is a lane for mRNA of the hepatic cancer 48 hours after DEN administration, Lane 5 is a lane for mRNA of the hepatic cancer 1 month after DEN administration, Lane 6 is a lane for mRNA of the hepatic cancer 3 months after DEN administration, Lane 7 is a lane for mRNA of the hepatic cancer 5 months after DEN administration, Lane 8 is a lane for mRNA of the hepatic cancer 7 months after DEN administration, and arrow 9 shows the position of the band of GADII mRNA.

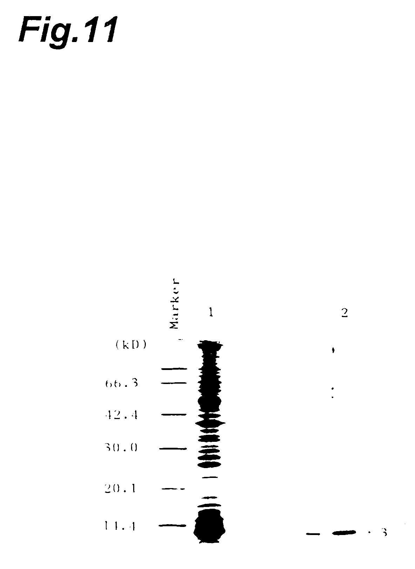

Fig. 11 is a view showing the results of SDS-PAGE electrophoresis of a recombinant HRPI partial protein. Lane 1 is a lane for a protein prepared by recombinant E. coli. Lane 2 is a lane for purified recombinant HRPI partial protein. An arrow 3 represents the position of the band of the HRPI partial protein.

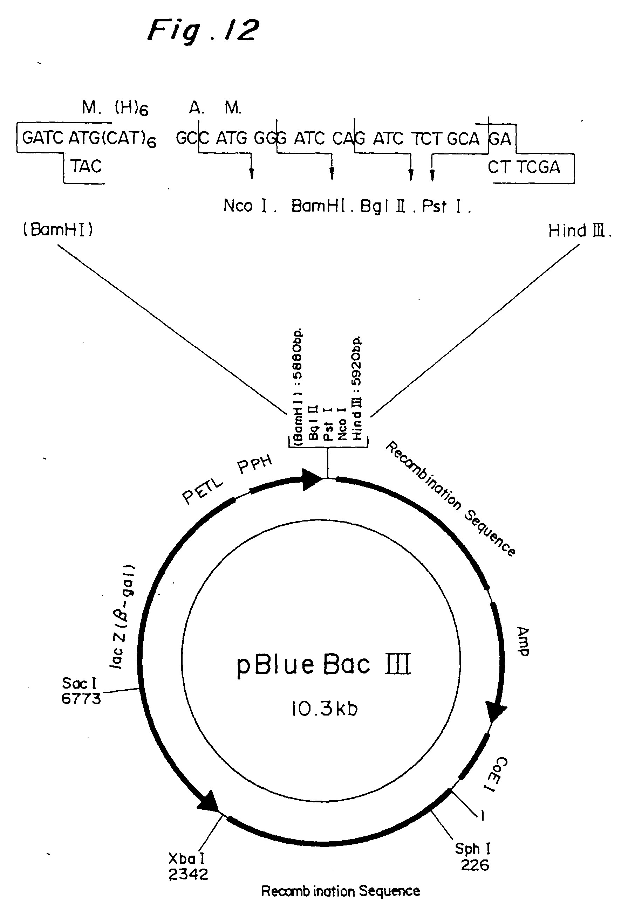

Fig. 12 is a view showing pBluebacIII vector as used in Example 15.

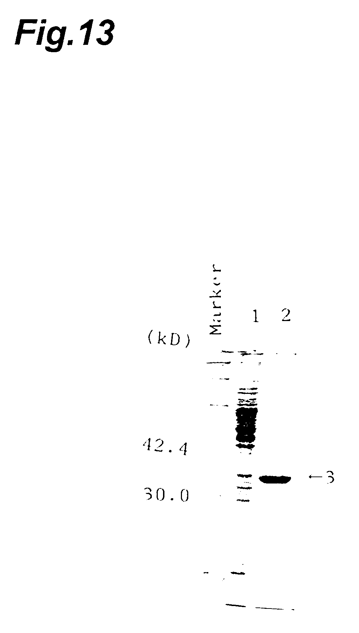

Fig. 13 is a view showing the results of SDS-PAGE electrophoresis of recombinant HRPI. Lane 1 is a lane for a protein expressed by wild type Sf9 cells. Lane 2 is a lane for a protein expressed by recombinant Sf9 cells. An arrow 3 represents the position of the band of the recombinant HRPI.

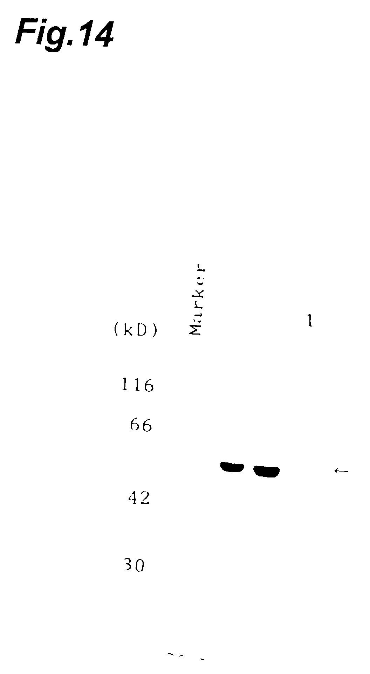

Fig. 14 is a view showing the results of 10%SDS-PAGE electrophoresis of recombinant GADII (Lane 1). An arrow represents the position of the band of the recombinant GADII.

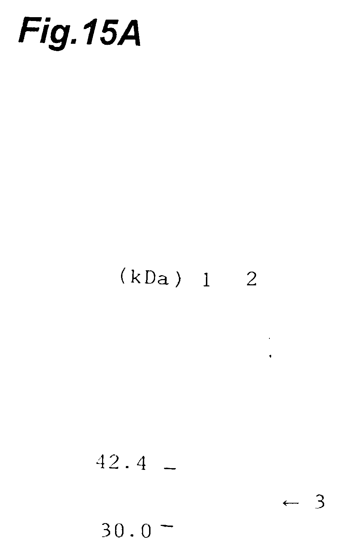

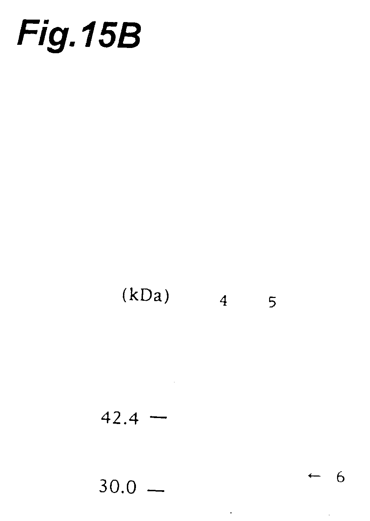

Figs. 15A and 15B are views showing the results of Western blot analysis of the reaction product between a sample containing HRPI full length protein and anti-HRPI antibodies. Fig. 15A is a view showing the results of Western blot analysis of the reaction product between a sample containing recombinant HRPI full length protein and anti-HRPI antibodies. Fig. 15B is a view showing the results of Western blot analysis of the reaction product between the extract of a rat liver and anti-HRPI antibodies. In Fig. 15A, lane 1 is a lane for the electrophoresed recombinant HRPI, lane 2 is a lane for the electrophoresed hepatic cancer tissue extract, and an arrow 3 represents the position of the band of the recombinant HRPI. In Fig. 15B, lane 4 is a lane for the electrophoresed normal liver extract, lane 5 is a lane for the electrophoresed extract of hepatic cancer 7 months after administration of DEN, and an arrow 6 represents the position of the band of HRPI.

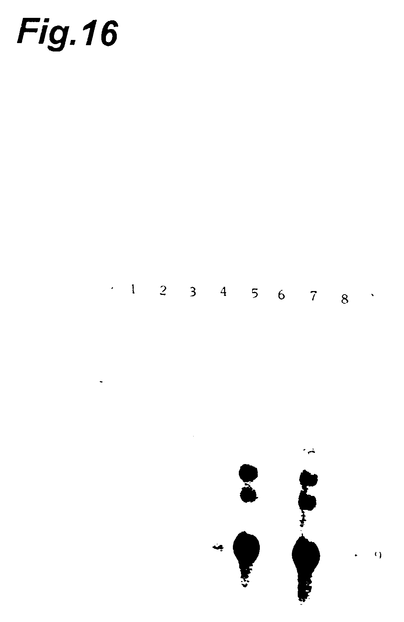

Fig. 16 is a view showing the expression of HRPI gene in various organs. Lane 1 is a lane for electrophoresed heart mRNA, lane 2 is a lane for electrophoresed brain mRNA, lane 3 is a lane for electrophoresed spleen mRNA, lane 4 is a lane for electrophoresed lung mRNA, lane 5 is a lane for electrophoresed liver mRNA, lane 6 is a lane for electrophoresed skeletal muscle mRNA, lane 7 is a lane for electrophoresed kidney mRNA, lane 8 is a lane for electrophoresed testicle mRNA, and an arrow 9 represents the position of the band of HRPI mRNA.

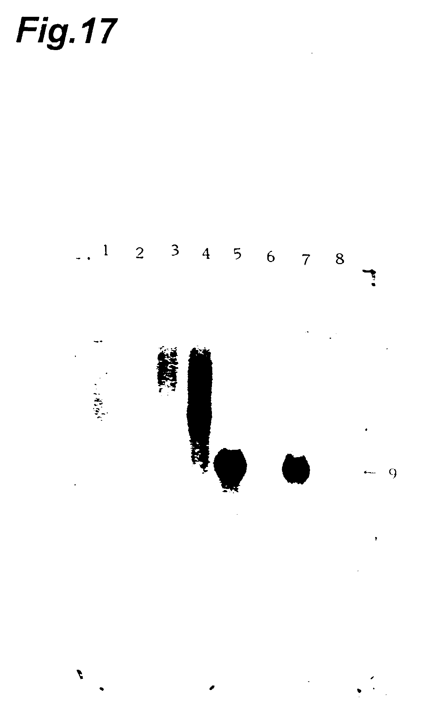

Fig. 17 is a view showing the expression of GADII gene in various organs. Lane 1 is a lane for electrophoresed heart mRNA, lane 2 is a lane for electrophoresed brain mRNA, lane 3 is a lane for electrophoresed spleen mRNA, lane 4 is a lane for electrophoresed lung mRNA, lane 5 is a lane for electrophoresed liver mRNA, lane 6 is a lane for electrophoresed skeletal muscle mRNA, lane 7 is a lane for electrophoresed kidney mRNA, lane 8 is a lane for electrophoresed testicle mRNA, and an arrow 9 represents the position of the band of GADII mRNA.

### Best Mode for Carrying Out the Invention

The present invention concerns proteins whose expression is increased in a hepatic cancer tissue compared with a normal hepatic tissue, genes encoding these proteins, antibodies specific for the proteins, and a method for monitoring cancer by detecting the proteins or the genes.

The characteristics of the present invention are as follows:
1. A protein represented by an amino acid sequence described as Sequence (Seq.) ID No. 1 in the Sequence Listing.
2. A protein represented by an amino acid sequence described as Sequence (Seq.) ID No. 1 in the Sequence Listing in which one or more amino acids has been or have been added, deleted or replaced, and whose expression in a hepatic tissue is increased in cancer cells compared with normal cells.
3. The protein recited in the section 2, in which mRNA encoding the protein is increased in cancer cells compared with normal cells at a level detectable by the subtraction method.
4. DNA containing a base sequence encoding the protein recited in any one of the sections 1 to 3.
5. DNA which contains a base sequence encoding at least part of the protein recited in any one of the sections 1 to 3, and which hybridizes with RNA encoding all of the protein.
6. DNA containing a base sequence described as Seq. ID No. 2 in the Sequence Listing.
7. DNA containing a base sequence ranging from the 500th base A to the 2520th base A in the base sequence described as Seq. ID No. 2 in the Sequence Listing.
8. DNA containing a base sequence ranging from the 515th base A to the 1315th base C in the base sequence described as Seq. ID No. 2 in the Sequence Listing.
9. DNA containing a base sequence ranging from the 1st base C to the 499th base T in the base sequence described as Seq. ID No. 2 in the Sequence Listing.
10. DNA which comprises consecutive 12 bases or more in a base sequence encoding the protein recited in any one of the sections 1 to 3, and whose GC content is 30 to 70%.
11. DNA which comprises consecutive 16 bases or more in a base sequence encoding the protein recited in any one of the sections 1 to 3, and whose GC content is 30 to 70%.
12. DNA which comprises consecutive 12 bases or more in the base sequence described as Seq. ID No. 2 in the Sequence Listing, and whose GC content is 30 to 70%.
13. DNA which comprises consecutive 16 bases or more in the base sequence described as Seq. ID No. 2 in the Sequence Listing, and whose GC content is 30 to 70%.
14. The DNA recited in any one of the sections 4 to 13, which has been chemically modified.
15. Antisense DNA to the DNA recited in any one of the sections 4 to 13.
16. RNA containing a base sequence encoding the protein recited in any one of the sections 1 to 3.
17. RNA which comprises consecutive 12 bases or more in a base sequence encoding the protein recited in any one of the sections 1 to 3, and whose GC content is 30 to 70%.
18. RNA which comprises consecutive 16 bases or more in a base sequence encoding the protein recited in any one of the sections 1 to 3, and whose GC content is 30 to 70%.
19. The RNA recited in any one of the sections 16 to 18, which has been chemically modified.
20. Antisense RNA to the RNA recited in any one of the sections 16 to 18.
21. Antibodies specifically reactive with the protein recited in any one of the sections 1 to 3.
22. The antibodies of the section 21 which react with human CRTI and rat CRTI.
23. A method for detecting the protein recited in any one of the sections 1 to 3, the method using the antibodies recited in the section 21 or 22.
24. A method for detecting cancer, comprising detecting the protein recited in any one of the sections 1 to 3, which is present in a tissue of a mammal, by use of the antibodies recited in the section 21 or 22.
25. The method for detecting cancer recited in the section 24, in which the tissue of the mammal is a hepatic tissue.
26. A method for detecting RNA encoding the protein recited in any one of the sections 1 to 3, the method using the DNA recited in any one of the sections 4 to 14 as a probe.
27. A method for detecting cancer, comprising detecting RNA encoding the protein recited in any one of the sections 1 to 3, which is present in a tissue of a mammal, by use of the DNA recited in any one of the sections 4 to 14 as a probe.
28. The method for detecting cancer recited in the section 27, in which the tissue of the mammal is a hepatic tissue.
29. A protein represented by an amino acid sequence described as Seq. ID No. 3 in the Sequence Listing.
30. A protein represented by an amino acid sequence described as Seq. ID No. 3 in the Sequence Listing, in which one or more amino acids has been or have been added, deleted or replaced, and whose expression in a hepatic tissue is increased in cancer cells compared with normal cells.
31. The protein recited in the section 30, in which mRNA encoding the protein is increased in cancer cells compared with normal cells at a level detectable by the subtraction method.
32. DNA containing a base sequence encoding the protein recited in any one of the sections 29 to 31.
33. DNA which contains a base sequence encoding at least part of the protein recited in any one of the sections 29 to 31, and which hybridizes with RNA encoding all of the protein.
34. DNA containing a base sequence described as Seq. ID No. 4 in the Sequence Listing.
35. DNA containing a base sequence ranging from the 25th base A to the 924th base G in the base sequence described as Seq. ID No. 4 in the Sequence Listing.
36. DNA which comprises consecutive 12 bases or more in a base sequence encoding the protein recited in any one of the sections 29 to 31, and whose GC content is 30 to 70%.
37. DNA which comprises consecutive 16 bases or more in a base sequence encoding the protein recited in any one of the sections 29 to 31, and whose GC content is 30 to 70%.
38. DNA which comprises consecutive 12 bases or more in the base sequence described as Seq. ID No. 4 in the Sequence Listing, and whose GC content is 30 to 70%.
39. DNA which comprises consecutive 16 bases or more in the base sequence described as Seq. ID No. 4 in the Sequence Listing, and whose GC content is 30 to 70%.
40. The DNA recited in any one of the sections 32 to 39, which has been chemically modified.
41. Antisense DNA to the DNA recited in any one of the sections 32 to 39.
42. RNA containing a base sequence encoding the protein recited in any one of the sections 29 to 31.
43. RNA which comprises consecutive 12 bases or more in a base sequence encoding the protein recited in any one of the sections 29 to 31, and whose GC content is 30 to 70%.
44. RNA which comprises consecutive 16 bases or more in a base sequence encoding the protein recited in any one of the sections 29 to 31, and whose GC content is 30 to 70%.
45. The RNA recited in any one of the sections 42 to 44, which has been chemically modified.
46. Antisense RNA to the RNA recited in any one of the sections 42 to 44.
47. Antibodies specifically reactive with the protein recited in any one of the sections 29 to 31.
48. A method for detecting the protein recited in any one of the sections 29 to 31, the method using the antibodies recited in the section 47.
49. A method for detecting cancer, comprising detecting the protein recited in any one of the sections 29 to 31, which is present in a tissue of a mammal, by use of the antibodies recited in the section 47.
50. The method for detecting cancer recited in the section 49, in which the tissue of the mammal is a hepatic tissue.
51. A method for detecting RNA encoding the protein recited in any one of the sections 29 to 31, the method using the DNA recited in any one of the sections 32 to 40 as a probe.
52. A method for detecting cancer, comprising detecting RNA encoding the protein recited in any one of the sections 29 to 31, which is present in a tissue of a mammal, by use of the DNA recited in any one of the sections 32 to 40 as a probe.
53. The method for detecting cancer recited in the section 52, in which the tissue of the mammal is a hepatic tissue.
54. A protein represented by an amino acid sequence described as Seq. ID No. 5 in the Sequence Listing.
55. A protein represented by an amino acid sequence described as Seq. ID No. 5 in the Sequence Listing, in which one or more amino acids has been or have been added, deleted or replaced, and whose expression in a hepatic tissue is increased in cancer cells compared with normal cells.
56. The protein recited in the section 55, in which mRNA encoding the protein is increased in cancer cells compared with normal cells at a level detectable by the subtraction method.
57. DNA containing a base sequence encoding the protein recited in any one of the sections 54 to 56.
58. DNA which contains a base sequence encoding at least part of the protein recited in any one of the sections 54 to 56, and which hybridizes with RNA encoding all of the protein.
59. DNA containing a base sequence described as Seq. ID No. 6 in the Sequence Listing.
60. DNA containing a base sequence ranging from the 65th base A to the 1582nd base A in the base sequence described as Seq. ID No. 6 in the Sequence Listing.
61. DNA which comprises consecutive 12 bases or more in a base sequence encoding the protein recited in any one of the sections 54 to 56, and whose GC content is 30 to 70%.
62. DNA which comprises consecutive 16 bases or more in a base sequence encoding the protein recited in any one of the sections 54 to 56, and whose GC content is 30 to 70%.
63. DNA which comprises consecutive 12 bases or more in the base sequence described as Seq. ID No. 6 in the Sequence Listing, and whose GC content is 30 to 70%.
64. DNA which comprises consecutive 16 bases or more in the base sequence described as Seq. ID No. 6 in the Sequence Listing, and whose GC content is 30 to 70%.
65. The DNA recited in any one of the sections 57 to 64, which has been chemically modified.
66. Antisense DNA to the DNA recited in any one of the sections 57 to 64.
67. RNA containing a base sequence encoding the protein recited in any one of the sections 54 to 56.
68. RNA which comprises consecutive 12 bases or more in a base sequence encoding the protein recited in any one of the sections 54 to 56, and whose GC content is 30 to 70%.
69. RNA which comprises consecutive 16 bases or more in a base sequence encoding the protein recited in any one of the sections 54 to 56, and whose GC content is 30 to 70%.
70. The RNA recited in any one of the sections 67 to 69, which has been chemically modified.
71. Antisense RNA to the RNA recited in any one of the sections 67 to 69.
72. Antibodies specifically reactive with the protein recited in any one of the sections 54 to 56.
73. The antibodies recited in the section 72, which are reactive with rat GADII.
74. A method for detecting the protein recited in any one of the sections 54 to 56, the method using the antibodies recited in the section 72 or 73.
75. A method for detecting cancer, comprising detecting the protein recited in any one of the sections 54 to 56, which is present in a tissue of a mammal, by use of the antibodies recited in the section 72 or 73.
76. The method for detecting cancer recited in the section 75, in which the tissue of the mammal is a hepatic tissue.
77. A method for detecting RNA encoding the protein recited in any one of the sections 54 to 56, the method using the DNA recited in any one of the sections 57 to 65 as a probe.
78. A method for detecting cancer, comprising detecting RNA encoding the protein recited in any one of the sections 54 to 56, which is present in a tissue of a mammal, by use of the DNA recited in any one of the sections 57 to 65 as a probe.
79. The method for detecting cancer recited in the section 78, in which the tissue of the mammal is a hepatic tissue.
80. A protein represented by an amino acid sequence described as Seq. ID No. 7 in the Sequence Listing.
81. A protein represented by an amino acid sequence described as Seq. ID No. 7 in the Sequence Listing, in which one or more amino acids has been or have been added, deleted or replaced, and whose expression in a hepatic tissue is increased in cancer cells compared with normal cells.
82. The protein recited in the section 81, in which mRNA encoding the protein is increased in cancer cells compared with normal cells at a level detectable by the subtraction method.
83. DNA containing a base sequence encoding the protein recited in any one of the sections 80 to 82.
84. DNA which contains a base sequence encoding at least part of the protein recited in any one of the sections 80 to 82, and which hybridizes with RNA encoding all of the protein.
85. DNA containing a base sequence described as Seq. ID No. 8 in the Sequence Listing.
86. DNA containing a base sequence ranging from the 72nd base A to the 1550th base G in the base sequence described as Seq. ID No. 8 in the Sequence Listing.
87. DNA which comprises consecutive 12 bases or more in a base sequence encoding the protein recited in any one of the sections 80 to 82, and whose GC content is 30 to 70%.
88. DNA which comprises consecutive 16 bases or more in a base sequence encoding the protein recited in any one of the sections 80 to 82, and whose GC content is 30 to 70%.
89. DNA which comprises consecutive 12 bases or more in the base sequence described as Seq. ID No. 8 in the Sequence Listing, and whose GC content is 30 to 70%.
90. DNA which comprises consecutive 16 bases or more in the base sequence described as Seq. ID No. 8 in the Sequence Listing, and whose GC content is 30 to 70%.
91. The DNA recited in any one of the sections 83 to 90, which has been chemically modified.
92. Antisense DNA to the DNA recited in any one of the sections 83 to 90.
93. RNA containing a base sequence encoding the protein recited in any one of the sections 80 to 82.
94. RNA which comprises consecutive 12 bases or more in a base sequence encoding the protein recited in any one of the sections 80 to 82, and whose GC content is 30 to 70%.
95. RNA which comprises consecutive 16 bases or more in a base sequence encoding the protein recited in any one of the sections 80 to 82, and whose GC content is 30 to 70%.
96. The RNA recited in any one of the sections 93 to 95, which has been chemically modified.
97. Antisense RNA to the RNA recited in any one of the sections 93 to 95.
98. Antibodies specifically reactive with the protein recited in any one of the sections 80 to 82.
99. The antibodies recited in the section 98, which are reactive with human GADII.
100. A method for detecting the protein recited in any one of the sections 80 to 82, the method using the antibodies recited in the section 98 or 99.
101. A method for detecting cancer, comprising detecting the protein recited in any one of the sections 80 to 82, which is present in a tissue of a mammal, by use of the antibodies recited in the section 98 or 99.
102. The method for detecting cancer recited in the section 102, in which the tissue of the mammal is a hepatic tissue.
103. A method for detecting RNA encoding the protein recited in any one of the sections 80 to 82, the method using the DNA recited in any one of the sections 83 to 91 as a probe.
104. A method for detecting cancer, comprising detecting RNA encoding the protein recited in any one of the sections 80 to 82, which is present in a tissue of a mammal, by use of the DNA recited in any one of the sections 83 to 91 as a probe.
105. The method for detecting cancer recited in the section 104, in which the tissue of the mammal is a hepatic tissue.

The present invention is a protein specifically expressed in hepatic cancer, and a gene encoding the protein. As disclosed in the Examples to be described later on, the protein and the gene were obtained by isolating a gene, present specifically in hepatic cancer, from the liver of a rat by the subtraction method, and determining its base sequence. From this gene, a protein expressed specifically in hepatic cancer was confirmed and isolated.

In this manner, the inventors identified and isolated four proteins and four genes newly. These proteins specific for hepatic cancer were named rat CRTI, rat HRPI, rat GADII and human GADII, respectively. The amino acid sequences of these proteins, and gene sequences encoding them are shown in Seq. ID Nos. 1 and 2, 3 and 4, 5 and 6, and 7 and 8, respectively, in the Sequence Listing.

Heterologous proteins with the same function are generally known to have homology of 30-40% or more, although they are somewhat different in amino acid sequence depending on species. It can be fully presumed, therefore, that mammals other than rat also have a protein having the same function as the protein isolated in the present invention (CRTI or HRPI), and having homology of 30% or more (preferably 40% or more) to this protein. The presence of homology, referred to herein, is generally determined by a calculation method which counts homologous amino acids as positive. When two proteins are different in the length of the amino acid chain, the homology rate (%) is expressed as the proportion of the homologous portion to the length of the protein with the shorter amino acid chain.

Thus, the proteins relevant to the present invention are intended to mean those having the following two features:
(1) In a hepatic tissue, expression in hepatic cancer is markedly increased compared with expression in normal cells.
(2) There is homology of 30% or more, preferably 40% or more, to the base sequence described in the Sequence Listing. Furthermore, this feature does not depend on the species of origin.

To know whether the protein is specifically observed (expressed) in hepatic cancer, various known methods are usable. For example, those in the Examples to be described later on are preferred methods.

Spontaneous or artificial mutation (see, e.g., 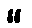 Molecular Cloning 2nd Edition 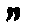 , Cold Spring Harbor Laboratory Press, 1989) pp. 15.1-15.113) can alter part of the structure of a polynucleotide, and accordingly, can mutate a protein encoded by the polynucleotide. In regard to the protein of the present invention, too, it is possible to produce a mutant protein in which one or more amino acids of the amino acid sequence described as Seq. ID No. 1, 3, 5 or 7 in the Sequence Listing has or have been replaced, deleted or added. The protein of the present invention is recognized specifically by antibodies to the protein, so that the mutant protein of the present invention is characterized by being recognized by the antibodies (being mutated to a degree recognizable by the antibodies).

The genes encoding CRTI, HRPI and GADII concerned with the present invention are genes coding for the CRTI, HRPI and GADII defined earlier.

The present invention clarifies the antigenicity of the above-mentioned proteins, i.e. CRTI, HRPI and GADII, such that these proteins easily give antibodies when used to immunize mammals other than the species of origin and other than human, as will be exemplified in the Examples. Hence, the antibodies of the present invention against CRTI, HRPI and GADII include, in their range, antisera and polyclonal antibodies which are obtained by the same method, namely, immunization of an animal other than the species from which the proteins were derived. The use, as an immunogen, of part of the protein or part of the protein bound to a carrier protein such as bovine serum albumin is a method in common use by those skilled in the art. The part of the protein may be synthesized by a peptide synthesizer. Preferably, the part of the protein is 8 amino acid residues or more for sufficient use as an immunogen.

If polyclonal antibodies against the substances whose antigenicity has been elucidated are obtained by immunization, monoclonal antibodies can be produced by hybridomas using lymphocytes of the immunized animal (e.g., see 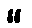 Antibodies, A Laboratory Manual 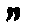 (Cold Spring Harbor Laboratory Press, 1988), Chapter 6). Thus, the antibodies of the present invention to CRTI, HRPI and GADII include polyclonal antibodies and monoclonal antibodies in their scope.

Methods for detecting CRTI, HRPI or GADII include, for example, the use of antibodies, the use of antibodies relying on an enzyme reaction, and the detection of the gene for the protein.

Methods using antibodies are concretely (1) a method of detecting each protein by use of antibodies to the protein, and (2) a method of detecting each protein by using antibodies to the protein and labeled secondary antibodies to the antibodies. The labels are, for instance, radioisotopes (RI), enzymes, avidin or biotin, and fluorescent materials (FITC or rhodamine).

Methods using antibodies relying on an enzyme reaction include, for example, ELISA.

Methods for detecting genes are, for instance, Northern blot hybridization, RT-PCR ( 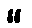 Current Protocols in Molecular Biology 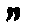 (Greene Publishing Associates and Wiley-Interscience), Chapter 15), and in situ hybridization (ibid. Chapter 14).

The optimum conditions for hybridization are known to be different according to the length of the probe or the membrane used. What conditions to be set can be easily chosen by those skilled in the art.

The Examples of the present invention disclose optimum conditions worked out in view of the nature of the membrane used and the length of the probe aimed at. If the membrane and the probe length differ, hybridization, needless to say, can be achieved under different hybridization conditions. For instance, hybridization may be effected in the absence of sodium pyrophosphate. A preferred embodiment is as follows:

### (1) Prehybridization conditions

5 - 10 x SSC (the concentration of each component of SSC solution is 5 to 10 times)
5 - 10 x Denhardt's solution
Not more than 1M sodium pyrophosphate (pH 6.8)
30 - 50% formamide
0.1 - 1% SDS
About 100 µg/ml yeast tRNA
About 100 µg/ml thermally denatured DNA
Reaction temperature 35 to 42°C
Reaction time 50 minutes to 1 hour and 10 minutes

### (2) Hybridization conditions

5 - 10 x SSC
5 - 10 x Denhardt's solution
Not more than 1M sodium pyrophosphate (pH 6.8)
30 - 50% formamide
Not more than 1% SDS
About 100 µg/ml yeast tRNA
About 100 µg/ml thermally denatured DNA
1x10⁵ to 2x10⁶ cpm/ml cDNA probe
Reaction temperature 35 to 42°C
Reaction time 12 to 20 hours
   Of the above conditions, sodium pyrophosphate (pH 6.8) or SDS is mainly used for improvement based on the background. The probe for use in detecting these genes may be DNA or RNA.

The number of bases of the human genome is said to be 3x10⁹. Since DNA of 16 bases comes in 4¹⁶ types, this length of DNA enables human proteins to be all recognized.

That is, the length required of a probe is, theoretically, 16 bases. For practical purposes, a length longer than it is desirable needless to say. Practically, a length of 12 bases or more is often used because of synthesis efficiency, operability and so on. The site used as the probe may be a noncoding region or a coding region.

When the site used as the probe has a GC content of 30 to 70%, it may be a noncoding region or a coding region.

The antisense DNA or antisense RNA of the present invention (may be collectively called the antisense polynucleotide) includes all of nucleotides comprising bases, phosphates and sugars combined in a plurality of numbers, including those which are not present in nature.

The antisense polynucleotide derivative of the present invention includes all of those which are similar to polynucleotide in conformation and function. Examples are polynucleotides linked at the 3'-terminal or 5'-terminal to other substances, polynucleotides having undergone modification, such as replacement, deletion, or addition, in at least part of the bases, sugars or phosphates, polynucleotides having bases, sugars or phosphates that do not naturally occur, and polynucleotides having a skeleton other than sugar-phosphate skeleton.

The antisense polynucleotide or its derivative may be the one which hybridizes with any portion of a polynucleotide encoding the protein of the present invention whose expression is increased in hepatic cancer. The antisense polynucleotide or its derivative is preferably the one which has a base sequence complementary for part of mRNA encoding all or part of the protein, and which hybridizes with the mRNA. The particularly preferred one is that which hybridizes with mRNA encoding at least CRTI, HRPI or GADII.

The antisense polynucleotide or its derivative is immediately usable as a research polynucleotide probe for investigating the presence and the status of expression, in a tissue or cells, of a polynucleotide encoding the protein of the present invention whose expression is increased in hepatic cancer. The antisense polynucleotide or its derivative is also usable as a diagnostic polynucleotide probe. The probe preferably has 12 bases or more and a GC content of 30 to 70%, more preferably, 16 bases or more and a GC content of 30 to 70%.

It is also possible to adjust the expression of the protein of the present invention whose expression is increased in hepatic cancer, by using the antisense polynucleotide or its derivative. This antisense polynucleotide or derivative is expected to prevent the expression of the protein by hybridizing with a gene or mRNA encoding the protein. Thus, it can be used as a therapeutic agent for a disease based on the disorder of function which the protein takes part in. That is, an antisense pharmaceutical can be developed from the antisense polynucleotide or its derivative.

Generally, preferred examples of the antisense polynucleotide derivative are known to be the derivatives with at least one of nuclease resistance, tissue selectivity, cell permeation, and binding capacity. Particularly preferably, the polynucleotide derivative is known to be the derivative having a phosphorothioate bond as a skeletal structure. The polynucleotide and its derivative of the present invention also include derivatives having such function or structure.

The antisense polynucleotide derivative of the present invention can be prepared, for example, by the method described in 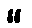 Antisense Research and Applications 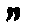 (CRC Publishing, Florida, 1993).

Wild type DNA or RNA, for instance, can be synthesized by a chemical synthesizer. Alternatively, the antisense polynucleotide derivative of the present invention can be obtained by PCR using as a template a gene encoding the protein of the invention whose expression is increased in hepatic cancer. Some of the derivatives of the methyl phosphonate type or the phosphorothioate type can be synthesized by a chemical synthesizer (e.g. Model 394, Perkin-Elmer Japan). In this case, the procedure is performed in accordance with an Instruction Manual attached to the chemical synthesizer, and the resulting synthesis product is purified by HPLC using a reversed phase chromatograph, whereby the desired polynucleotide or polynucleotide derivative can be obtained.

When DNA or RNA is chemically or enzymatically synthesized, it is well known to subject it to a chemical modification, such as methylation or biotinylation of the side chain, or substitution of oxygen of the phosphate portion by sulfur.

Main examples of the modification to be introducible during chemical synthesis are 1) biotinylation, 2) methylation, 3) conversion to digoxigenin derivative, 4) dephosphorylation, 5)fluorescence labeling (fluorescein, rhodamine, Texas Red and derivatives thereof), 6) amination, and 7) substitution of O of the phosphate group by S.

Main examples of chemical modification to be introducible enzymatically are 1) biotinylation, 2) methylation, 3) conversion to digoxigenin derivative, 4) dephosphorylation, 5)fluorescence labeling (fluorescein, rhodamine, Texas Red and derivatives thereof), and 6) enzyme labeling (alkaline phosphatase).

When DNA described as Seq. ID No. 2, 4, 6 or 8 in the Sequence Listing is to be chemically synthesized, for instance, it is possible to synthesize DNA different from the DNA of the Sequence Listing itself by performing the chemical modification. Thus, the DNA and RNA of the present invention include in their scopes the chemically modified DNA and RNA.

Monitoring of the progress of cancer by detection of CRTI, HRPI or GADII can be performed by investigating whether the protein is present or not in a tissue or cells sampled from subjects. If CRTI, HRPI or GADII is secreted or liberated outside cells, the progress of cancer can be monitored by examining whether CRTI, HRPI or GADII is present or absent in the blood of the subject. Concretely, the monitoring may be done in the same manner as the aforementioned method of detecting CRTI, HRPI or GADII (the method using antibodies, the method using antibodies relying on enzyme reaction, or the method detecting the relevant gene).

Monitoring of the progress of cancer by use of gene can be done by checking for the gene in a tissue or cells sampled from the subject. The methods of detecting the gene include, for example, Northern blot hybridization, RT-PCR, and in situ hybridization, as stated previously.

### Examples

The present invention will now be described in detail by reference to Examples, which do not limit the present invention.

### [Example 1] Protein increasing in expression in hepatic cancer and its gene

### I. Isolation of gene encoding protein whose expression is increased in hepatic cancer

### 1. Preparation of hepatic cancer rats

Hepatic cancer-bearing rats were prepared based on the Salt-Farber method ( 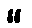 Nature 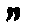 , Vol. 263, 1976, pp. 701-703). Actually, 5-week Wistar rats (Funabashi Farm) were intraperitoneally administered diethylnitrosamine (DEN). Two weeks later, oral administration of M Feed (Oriental Yeast) containing 0.02% of 2-aminoacetylfluorene (AAF) was started. One week later, a regenerative hepatic operation was performed. The livers were removed 12, 24, 48 hours and 1, 3, 5 and 7 months after DEN administration, and used for subsequent RNA preparation.

As controls, regenerated livers showing normal growth were acquired by removing the livers 12, 24 and 48 hours after regenerative hepatic operation. These livers were used later for analysis.

### 2. Preparation of RNA

Total RNA was prepared based on the method described in 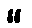 Methods in enzymology 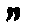 , Vol. 154 (Academic Press Inc., 1987), pp. 3-28. The actual procedure was as follows:
(1) Three grams of each liver was ground in liquid nitrogen, and added to 100 ml of a 5.5M GTC solution (5.5 mM guanidine thiocyanate, 0.5% N-lauronylsarcosine, 25 mM sodium citrate, pH 7.0). The mixture was homogenized with a Potter type homogenizer.
(2) The homogenate was centrifuged for 10 minutes at 3,000 rpm, and then the supernatant was placed on 12 ml of a cesiumtrifluoroacetic acid solution (50% cesiumtrifluoroacetic acid (Pharmacia), 100 mM disodium ethylenediaminetetraacetate (EDTA) (pH 7.0)) with a specific gravity of 1.6 g/ml that had been added in an SW28 swing rotor centrifuge tube (Beckman). The system was separated by the SW28 swing rotor for 24 hours at 25,000 rpm at 15°C.
(3) The precipitate was dissolved in 600 µl of 4M GTC solution, and centrifuged at 15,000 rpm, whereafter the solution portion was recovered. Then, 15 µl of 1M acetic acid and 450 µl ethanol were added, and the mixture was centrifuged for 10 minutes at 15,000 rpm, followed by recovering the precipitate.
(4) The precipitate was dissolved in a suitable amount (about 3 ml) of a TE solution (1 mM Tris-Cl (pH 7.5), 1 mM EDTA) (TE solution was added until the precipitate was dissolved). The solution was centrifuged at 15,000 rpm, and the solution portion was recovered.
(5) Phenol/chloroform in the same amount as the amount of the solution was mixed with the solution. The mixture was centrifuged for 10 minutes at 15,000 rpm, and the solution portion was recovered.
(6) To the solution, a 1/10 volume of 3M sodium acetate (pH 5.2) was added, and ethanol in a 2.5-fold amount was added. The mixture was allowed to stand for 20 minutes at -20°C, and then centrifuged at 15,000 rpm. The precipitate was washed with 70% ethanol, and then dried, dissolved in a suitable amount of TE solution, and stored at -80°C. From each liver, 5 - 7 mg of total RNA was obtained.
(7) PolyA RNA was prepared by use of oligotex dT30 super™ (Nippon Roche) in accordance with an instruction manual attached to it. The amount of the total RNA used was 1 mg per cycle, and polyA RNA was purified with 750 µl of oligotex dT30 super™. In each liver, about 20 µg of polyA RNA was obtained from 1 mg of the total RNA.

### 3. cDNA subtraction

This method was performed in accordance with the method of Hara et al. ( 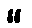 Analytical Biochemistry 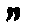 , Vol. 214, 1993, pp. 58-64 (this reference is a part of the instant specification as a result of its citation herein). The actual procedure was as follows:
(1) The polyA RNA used originated from each of the 7-month hepatic cancer and the normal liver, and 15 µg each was used.
(i) The polyA RNA's were each adsorbed by oligotex dT30 super™, and then the synthesis of cDNA was performed in this state. The conditions for the synthetic reaction followed the literature.
(ii) To the cDNA-oligotex dT30 super™ from hepatic cancer, poly dC tail was added using terminal deoxytransferase (TAKARA SHUZO), and cDNA for a sense strand was synthesized by means of EcoRI-(dG)₁₅ primer and Taq polymerase (Perkin-Elmer).
Between the sense strand cDNA and the cDNA-oligotex dT30 super™ from the normal liver, the cDNA subtraction reaction was carried out.
(iii) After the reaction, the resulting cDNA solution was amplified by a PCR reaction using both of primers, EcoRI-(dG)₁₅ and XhoI-(dT)₃₀, in accordance with the method described in 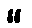 Current Protocols in Molecular Biology 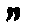 (1987, Greene Publishing Associates and Wiley-Interscience), Chapter 15. The conditions for the PCR were 25 cycles, each cycle comprising three stages at different temperatures, 94°C for 90 seconds, then 55°C for 2 minutes, and then 72°C for 3 minutes, using the solution of the following composition:

| | |
|---|---|
| cDNA solution | 69 µl |
| 10xTaq buffer (Perkin-Elmer) | 10 µl |
| 1.25 mM dNTP | 16 µl |
| EcoRI-(dG)₁₅ primer (2 µg/µl) | 2 µl |
| XhoI-(dT)₃₀ primer (2 µg/µl) | 2 µl |
| Taq polymerase (Perkin-Elmer)(5 µ/µl) | 1 µl |
| | Total 100 µl |

(2)
(i) After subtraction treatment, the resulting gene library was digested with EcoRI (TAKARA SHUZO). The reaction system employed the following conditions:

| | |
|---|---|
| Gene solution | 10 µl |
| 10xH buffer (TAKARA SHUZO) | 10 µl |
| EcoRI (TAKARA SHUZO) | 5 µl |
| Sterilized water | 75 µl |
| | Total 100 µl |

Reaction temperature 37°C.
Reaction time: Overnight

(ii) After cleavage with EcoRI, 100 µl phenol/chloroform was mixed with EcoRI digested gene library, and the mixture was centrifuged at 15,000 rpm, followed by recovering the aqueous solution portion. To the solution, 10 µl 3M sodium acetate (pH 5.2) was added, and 250 µl ethanol was further added. Then, the mixture was centrifuged at 15,000 rpm, and the precipitate was recovered.
(iii) The recovered precipitate was washed with 1 ml of 70% ethanol, dried, and then dissolved in 75 µl of sterilized water. The solution was formed into the following composition, which was reacted overnight at 37°C for cleavage with XhoI:

| | |
|---|---|
| Gene solution | 75 µl |
| 1% BSA (TAKARA SHUZO) | 10 µl |
| 10xH buffer (TAKARA SHUZO) | 10 µl |
| XhoI (TAKARA SHUZO) | 5 µl |
| | Total 100 µl |

Reaction temperature 37°C.
Reaction time: Overnight

(3) Then, 100 µl phenol/chloroform was mixed with the system, and the mixture was centrifuged at 15,000 rpm, followed by recovering the aqueous solution portion. To the solution, 10 µl 3M sodium acetate (pH 5.2) was added, and 250 µl ethanol was further added. Then, the mixture was centrifuged at 15,000 rpm, and the precipitate was recovered. The precipitate was washed with 1 ml of 70% ethanol, dried, and then dissolved in 100 µl of sterilized water to prepare a gene library solution.
4. Incorporation of gene after subtraction into vector
(1) pBluescriptII vector (Stratagene) was digested with EcoRI and XhoI (TAKARA SHUZO). The digesting conditions were the same as in 3.(2).
(2) The digested ends of the digested pBluescriptII vector were dephosphorylated by bacterial alkaline phosphatase for 1 hour at 65°C and then 100µl of phenol/chloroform was mixed with the system, and the mixture was centrifuged at 15,000 rpm, followed by recovering the aqueous solution portion.
(3) To the solution, 10 µl 3M sodium acetate (pH 5.2) was added, and 250 µl ethanol was further added. Then, the mixture was centrifuged at 15,000 rpm, and the precipitate was recovered. The precipitate was washed with 1 ml of 70% ethanol, dried, and then dissolved in sterilized water to a concentration of 100 ng/µl.
(4) The gene library solution obtained in 3. and the digested, dephosphorylated pBluescriptII vector were mixed and reacted in accordance with an instruction manual attached to ligation pack™ (Nippon Gene) to insert each gene of the library into the vector.

5. Incorporation of gene after subtraction into E. coli
In accordance with a customary method, the reaction mixture in 4.(4) was wholly mixed with competent cells of E. coliJM109 (TAKARA SHUZO), and reacted for 30 minutes on ice, for 45 seconds at 42°C, and for 3 minutes on ice. Then, 900 µl SOC culture medium was added, and the mixture was allowed to stand for 1 hour at 37°C to incorporate the vector into the E. coliJM109. Then, the E. coliJM109 was recovered.
6. Extraction of gene
(1) This E. coli was sprinkled over LB agar medium with the following supplements composition, and cultured overnight to form colonies.
Supplements composition of LB agar culture medium:

| | |
|---|---|
| Ampicillin (Wako Pure Chemical) | 100 µg/ml |
| IPTG (TAKARA SHUZO) | 0.1 mM |
| X-gal (TAKARA SHUZO) | 0.004% |

Of the colonies formed, white colonies were selected as inoculum for use in gene screening to be performed later on.
(2) 2,000 kinds of inocula were selected, and were each cultured in a 2 ml LB liquid medium containing 100 µg/ml ampicillin. Then, genes were extracted by the alkali method described in 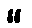 Molecular Cloning Second Edition 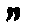 (Cold Spring Harbor Laboratory Press, 1989), Chapter 1.
7. Dot blot screening
(1) The extracted genes were each blotted to two nylon membranes (Millipore) using Bio Dot (Bio-Rad), and denatured with following condition.
   (i) The genes were each reacted with a solution of 0.1M sodium hydroxide and 0.15M sodium chloride for 20 seconds.
   (ii) Then, the system was reacted with a solution of 0.2M Tris-Cl (pH 7.5) and 0.15M sodium hydroxide for 2 minutes.
   (iii) Then, the system was reacted in 2xSSC for 2 minutes.

   The denatured genes were immobilized by a UV cross-linker (Stratagene)
(2) The hepatic cancer polyA RNA and normal liver polyA RNA prepared in 2. were subjected to a reverse transcription reaction with AMV reverse transcriptase (Seikagaku Kogyo) using radioactive CTP (α-³²P-dCTP) (Amersham) to prepare cDNA probes from the respective polyA RNA's.
(3) The genes fixed to the nylon membranes in
   (1) were each hybridized with each of the cDNA's prepared in (2) under the following conditions:
      (i) Prehybridization conditions
         5 x SSC
         5 x Denhardt's solution
         0.1M sodium pyrophosphate (pH 6.8)
         50% formamide
         0.5% SDS
         100 µg/ml yeast tRNA
         100 µg/ml denatured salmon sperm DNA
         Reaction temperature 42°C
         Reaction time 1 hour
      (ii) Hybridization conditions
         5 x SSC
         5 x Denhardt's solution
         0.1M sodium pyrophosphate (pH 6.8)
         50% formamide
         0.5% SDS
         100 µg/ml yeast tRNA
         100 µg/ml denatured salmon sperm DNA
         cDNA probe prepared in (2) (5x10⁵ cpm/ml)
         Reaction temperature 42°C
         Reaction time 16 hours
   (2) Then, the nylon membranes were each washed with 500 ml of 2 x SSC (containing 0.1% SDS), 0.2 x SSC and 0.1 x SSC in this order, each for 30 minutes at 60°C, and then subjected to autoradiography. The results are shown in Fig. 1. Based on the resulting autoradiograms, selection was made of the genes bound in larger amounts to the hepatic cancer-derived cDNA probe than to the normal liver-derived cDNA probe. At least 31 were confirmed in all. Three out of them were selected, and used for the following experiments:
8. Base sequencing
   Determination of the base sequence was performed in accordance with the method described in 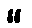 Molecular Cloning Second Edition 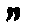 , Chapter 13. Actually, the base sequencing of the genes bound in large amounts to the hepatic cancer cDNA probe was carried out by reading the sequence of the gene portion inserted onto the pBluescriptII by the dideoxyterminator method using T7sequence kit™ (pharmacia).
9. Analysis of homology
   The determined base sequences of the genes were analyzed for homology by referring to the data bank of DDBJ (DNA Data Base of Japan). As a result, these three genes were found to be novel genes without homology. These genes were named CRTI gene, HRPI gene and GADII gene. The respective genes were analyzed in the following manner to know whether their lengths were full lengths:
10. Construction of cDNA library
   From 4 µg of the polyA RNA extracted from the hepatic cancer tissue 7 months after DEN administration, cDNA was synthesized by means of Pharmacia's Time Saver cDNA Synthesis Kit™ in accordance with its instruction manual. The outline of this synthesis is given below.
   (1) Reverse transcription reaction was performed using a random primer, and a 2nd-strand DNA synthesis reaction was carried out with a DNA polymerase to synthesize double-stranded cDNA. To add NotI/EcoRI adaptors to both ends of the cDNA, the cDNA was treated with T4 DNA ligase. Then ligated cDNA was phosphorelated with polynucleotide kinase. As a result, cDNA having EcoRI restriction enzyme cleavage sites at both ends was obtained.
   (2) The cDNA was inserted into λgt11 cloning vector (Pharmacia) by use of T4DNA ligase, and packaged with GIGAPACK Gold™ (Stratagene). Thus, the cDNA was incorporated into the λphage for isolation of the gene.
11. Isolation of gene
   Isolation of the gene was performed in accordance with the method described in 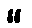 Molecular Cloning Second Edition 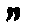 , Chapter 2. Its outline is offered below.
   (1) The cDNA-containing library constructed in 10. was contacted with Y1090r-E. coli, then mixed with NZY medium containing 0.7% agar, and sprinkled on NZY medium plate containing 1.5% agar. Incubation was performed for 6 hours at 42°C to form plaques containing large amounts of cDNA. Then, a nitrocellulose filter (Immobilon, Millipore's registered trademark, Millipore) was placed on the plate to transfer the plaques onto the filter.
   (2) The filter was treated with sodium hydroxide to denature the cDNA in the plaques. The conditions for denaturation were the same as described in 7.(1).
   (3) The denatured cDNA was heat-treated for 2 hours at 75°C to immobilize it, and was used for hybridization. The probe used in the hybridization was a part of the CRTI gene whose base sequence had been determined in 8., that part being randomly labeled with ³²P-dCTP by means of a random labeling kit of Boehringer Mannheim. The conditions for hybridization and the conditions for washing followed the conditions described in the literature.
   (4) As a result of hybridization, a gene having a longer sequence than the sequence of the probe was obtained from plaques corresponding to positive signals obtained from cDNA fixed to the filter.

   The foregoing procedure was performed for each of the three genes, CRTI gene, HRPI gene and GADII gene, to obtain the corresponding full-length genes.

### [Example 2] Determination of amino acid sequence of hepatic cancer-specific protein and gene encoding the protein

1. Large-scale preparation of gene
The following procedure was performed on CRTI gene to prepare the gene on a large scale:
(1) The λ phage recovered from the plaques formed on the NZY agar medium in 11.(4) or (5) of Example 1 was suspended in SM solution.
(2) 50 µl of the suspension in (1) and 20 µl of Y1090r-E. coli were mixed, and allowed to stand for 15 minutes at 37°C.
(3) Then, the mixed solution in (2) was transferred to 10 ml NZY medium containing 100 µg/ml ampicillin, and cultured for 6 hours at 37°C.
(4) The culture was centrifuged for 5 minutes at 8,000 rpm, and the supernatant was recovered.
(5) To the supernatant, 1 ml of 5M NaCl and 1.1 g of polyethylene glycol 6000 were added and dissolved.
(6) The solution was placed on ice for 1 hour, and then centrifuged for 20 minutes at 4°C at 10,000 rpm.
(7) The precipitate was recovered and suspended in 700 µl SM solution.
(8) 500 µl chloroform was added to the suspension, and the mixture was stirred to dissolve the remaining E. coli cells.
(9) The solution was centrifuged for 10 minutes at 5,000 rpm, and the aqueous phase was recovered.
(10) To the aqueous phase, 1 mg/ml RNaseA and 5 mg/ml DNaseI (both Sigma) were added in an amount of 1 µl each. After the mixture was left to stand at 37°C for 1 hour, 600 µl of 20% polyethylene glycol 6000 (0.8 M NaCl) was added. The mixture was allowed to stand for 30 minutes on ice.
(11) The system was centrifuged for 20 minutes at 4°C at 15,000 rpm, and then the precipitate was recovered.
(12) To the precipitate, 500 µl SM solution, 50 µl 5M NaCl and 50 µl 0.5M EDTA were added, and 400 µl phenol was further added. The mixture was stirred to dissolve the phage and liberate cDNA.
(13) The solution was centrifuged for 5 minutes at 15,000 rpm at room temperature, and then the aqueous phase was recovered. To the liquid, 1 ml ethanol was added, and the mixture was centrifuged for 20 minutes at 15,000 rpm. The liquid phase was discarded.
(14) The precipitate was washed with 1 ml of 70% ethanol, and dissolved in 100 µl TE solution (10 mM Tris-Cl pH 8.0, 1 mM EDTA) to obtain DNA solution.

2. Insertion of CRTI gene into vector
CRTI gene was inserted into a vector by the following procedure:
(1) A DNA cleavage system of the following composition was prepared to cut DNA with the restriction enzyme NotI (TAKARA SHUZO):

| | |
|---|---|
| DNA solution (prepared in 1.) | 20 µl |
| 0.1% BSA | 10 µl |
| 0.1% TritonX100 | 10 µl |
| NotI (TAKARA SHUZO) | 2 µl |
| Rnase (Nippon Gene) | 1 µl |
| 10xH buffer (Takara) | 10 µl |
| Sterilized water | 47 µl |
| | Total 100 µl |

Reaction temperature 37°C Reaction time 4 hours

(2) Then, the system was electrophoresed on 0.7% NuSieve™GTG Agarose (TAKARA SHUZO), and DNA of about 2.0 kbp was cut out. This DNA was recovered by means of GENE CLEAN II™ (Funakoshi) as in its instruction manual.
(3) pBluescriptII (Stratagene) to hold DNA was cleaved with NotI, and then dephosphorylated.
(i) Cleavage with NotI was carried out using the following system:

| | |
|---|---|
| pBluescriptII (1 µg/µl) | 3 µl |
| 10xH buffer | 2 µl |
| 0.1% BSA | 2 µl |
| 0.1% TritonX100 | 2 µl |
| NotI | 2 µl |
| Sterilized water | 10 µl |
| | Total 20 µl |

Reaction temperature 37°C
Reaction time: Overnight

(ii) Then, 2 µl of 1M Tris pH 8.0 was added, and 1 µl Bacterial Alkaline Phosphatase (TAKARA SHUZO) was added, followed by leaving the mixture to stand for 1 hour at 65°C.
(iii) Then, phenol/CHCl₃ extraction was performed twice in accordance with a customary manner to deactivate the enzymes. After purification by ethanol precipitation, the precipitate was dissolved in TE solution to a concentration of 100 ng/µl.
(4) The DNA obtained in (2) and the pBluescriptII obtained in (3) were reacted using the following system to insert the DNA into the vector:

| | |
|---|---|
| DNA (prepared in (2)) | 5 µl |
| NotI-cleaved pBluescriptII (prepared in (3)) | 1 µl |
| 10-fold ligation buffer (Nippon Gene) | 2 µl |
| T4 ligase (Nippon Gene) Sterilized water | 1 µl 11 µl |
| | Total 20 µl |

Reaction temperature 16°C
Reaction time 2 hours

3. Incorporation of CRTI gene into E. coli
In accordance with a customary method, the reaction mixture of the vector fitted with CRTI gene prepared in 2. was wholly mixed with competent cells of E. coli HB101 (TAKARA SHUZO). The mixture was reacted for 30 minutes on ice, for 45 seconds at 42°C, and for 3 minutes on ice. Then, 900 µl of SOC culture medium was added, and the mixture was allowed to stand for 1 hour at 37°C to incorporate the vector into the E. coli HB101.
The recombinant E. coli having CRTI gene (CRTI-A) introduced therein was deposited November 21, 1995 at the National Institute of Bioscience and Human Technology (Address: 1-3, Higashi 1-chome, Tsukuba-shi, Ibaraki-ken, Japan), Agency of Industrial Science and Technology, Japan, with the accession number FERM P-15302. Then, the microorganism was transferred to international deposition on September 9, 1996 (Accession No. FERM BP-5658).
4. Base sequencing of gene
(1) The E. coliHB101 recovered in 3. was sprinkled on LB agar medium (containing 100 µm/ml ampicillin, 0.1 mM IPTG, 0.004% X-gal), and incubated for 16 hours at 37°C.
(2) Of the colonies formed, white colonies were inoculated into 2 ml LB medium (containing 100 µg/ml ampicillin), and cultured for 16 hours at 37°C.
(3) Then, the culture was centrifuged for 1 minute at 12,000 rpm to harvest the cells, and a plasmid DNA solution was recovered by means of Magic Miniprep™ (Promega).
(4) The recovered DNA was subjected to T7 Sequencing Kit (Pharmacia) to determine the complete base sequence. As a result, CRTI-A was found not to contain a full length gene. Thus, the acquisition of full length CRTI gene was attempted again by the method of Example 1, 11., using, as a probe, a sequence ranging from the 524th base to the 1415th base of Seq. ID No. 2 in the Sequence Listing, instead of a part of CRTI gene whose base sequence was determined in Example 1, 8. This alternative probe was randomly labeled with ³²P-dCTP by use of a random labeling kit of Boehringer Mannheim. The conditions for hybridization and the conditions for washing followed the conditions described in the literature. A gene fragment obtained anew was introduced into E. coli by the same method as described in Example 2, 1., 2., and 3. The base sequence of the gene fragment was determined by the method described herein. Consequently, a CRTI gene fragment containing the 500th base to the 1415th base of Seq. ID No. 2 was obtained. The recombinant E. coli having this gene (CRTI-B) introduced therein was deposited November 21, 1995 at the National Institute of Bioscience and Human Technology (Address: 1-3, Higashi 1-chome, Tsukuba-shi, Ibaraki-ken, Japan), Agency of Industrial Science and Technology, Japan, with the accession number FERM P-15303. Then, the microorganism was transferred to international deposition on September 9, 1996 (Accession No. FERM BP-5659). Thus, there was obtained CRTI gene of 2,021 bp containing all of the coding region together with the CRTI-A acquired previously. CRTI-A covers the 525th base to the 2520th base of Seq. ID No. 2 in the Sequence Listing.

5. Determination of amino acid sequence
The amino acid sequence of CRTI was determined by the base sequence determined in 4. The amino acid sequence of CRTI is indicated as Seq. ID No. 1 in the Sequence Listing.

### [Example 3] Confirmation of possibility for detection (diagnosis) of hepatic cancer at gene expression level

1. Analysis of CRTI gene by Northern blot hybridization
   The analysis of CRTI gene by Northern blot hybridization was made in accordance with the method using formaldehyde denatured gel electrophoresis described in 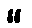 Current Protocols in Molecular Biology 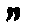 (1987, Greene Publishing Associates and Wiley-Interscience), Chapter 4. PolyA RNA used in the analysis was that derived from the rat normal liver, or that from the hepatic cancer 7 months after DEN administration in Example 1. The amount of the polyA RNA used was 500 ng for each sample.
   (1) The portion ranging from the 525th base to the 1,415th base as CRTI gene of Seq. ID No. 2 carried on pBluescriptII in Example 2 was cut out of the vector by treatment with a restriction enzyme.
   (2) Then, the restriction enzyme-treated liquor was subjected to agarose gel electrophoresis to separate the targeted CRTI gene (from the 525th base to the 1,415th base).
   (3) The CRTI gene separated in (2) was purified with GENE CLEAN II™ (Funakoshi).
   (4) The purified CRTI gene was labeled by Random Primed DNA Labeling Kit (Boehringer Mannheim) using α-P³²dCTP (Amersham) in accordance with its instruction manual to make a ³²P-dCTP-labeled CRTI gene probe.
   (5) Systems using the normal liver and the 7-month hepatic cancer were each analyzed by Northern blot hybridization using the probe prepared in (4). As a result, CRTI gene was detected as a gene significantly increased in hepatic cancer. This finding showed that hepatic cancer and normal liver could be distinguished, namely, hepatic cancer could be diagnosed, by using CRTI gene. The results are given in Fig. 2 (probe A). In autoradiography, the film was Kodak's XAR film, the screen used Du Pont's Lightening Plus, and exposure was performed for 24 hours at -80°C. In Fig. 2, HCC denotes hepatocellular carcinoma or hepatic cancer.

### [Example 4] Base sequencing of CRTI gene of 2.5 kb

CRTI gene was analyzed for hepatic cancer-derived polyA RNA by Northern blot hybridization using the probe of Example 3. As shown in Fig. 2 (probe A), two bands were detected. The band observed at about 2.0 kb corresponds to a gene of the base sequence from the 500th base to the 2,520th base of Seq. ID No. 2 in the Sequence Listing. A gene corresponding to the other band at about 2.5 kb was acquired by the method described in 11. of Example 1.

The base sequence of the resulting gene was determined by the method described in 1. to 4. of Example 2. This gene was found to be an extension of the gene of about 2.0 kb by 499 bp on the 5' side. The CRTI gene base sequence of the resulting portion of about 2.5 kb is shown as Seq. ID No. 2 in the Sequence Listing.

Recombinant E. coli having CRTI gene (R3) containing the 499 bp portion introduced therein (JM109) was deposited June 12, 1996 at the National Institute of Bioscience and Human Technology (Address: 1-3, Higashi 1-chome, Tsukuba-shi, Ibaraki-ken, Japan), Agency of Industrial Science and Technology, Japan, with the accession number FERM P-15685. Then, the microorganism was transferred to international deposition on September 9, 1996 (Accession No. FERM BP-5660).

Furthermore, Northern blot hybridization was performed in accordance with the method described in Example 3 by using, as a probe, DNA ranging from the 1st base to the 372nd base of the 499 bp portion obtained above. In autoradiography, the film was Kodak's XAR film, the screen used Du Pont's Lightening Plus(+), and exposure was performed for 24 hours at - 80°C. In systems using polyA RNA from a rat's normal liver and hepatic cancer, the presence of a band of 2.5 kb specific for rat hepatic cancer was confirmed (Fig. 2 (probe B)). This result showed that hepatic cancer could be detected based on the above-described DNA of 499 bp.

Homology search of the resulting CRTI through a database revealed TREB5 (or XBP) as a homologous sequence, which had homology of about 79% to CRTI of the present invention. TREB5 is reported in The EMBO Journal, vol. 9, No. 8, pp. 2537-2542 (1990), while XBP is reported in Science, vol. 247, pp. 1581-1584 (1990).

### [Example 5] Expression of CRTI protein

1. Construction of recombinant E. coli
   The CRTI-B gene obtained in Example 2 was integrated into pET3a vector having a histidine tag incorporated therein (Fig. 3), and was then introduced into E. coli BL21 (DE3) pLysS.
2. Preparation of CRTI
   (1) The E. coli produced in 1. was cultured in LB medium containing 100 µg/ml ampicillin. When turbidity measured with a spectrophotometer (Beckman) reached 0.5 at a wavelength of 600 nm, IPTG was added in a concentration of 0.5 mM to induce the expression of CRTI protein.
   (2) Two hours later, E. coli cells were recovered, and suspended in Lysis Buffer. The suspension was sonicated, and centrifuged for 15 minutes at 18,000 rpm at 4°C with 50.2 Ti rotor using Beckman Optima XL-80.
   (3) Then, the precipitate was dissolved in 6M guanidine hydrochloride, and purified with Ni-agarose (Qiagen) in accordance with its instruction manual.
3. SDS-PAGE electrophoresis
   The purified sample was subjected to SDS-polyacrylamide electrophoresis, and stained with Coomassie brilliant blue to make sure that purification was successful.

### [Example 6] Study of possibility for diagnosis of human hepatic cancer

(1) Total RNA was prepared from a human hepatic cancer tissue and a non-cancerous hepatic tissue of the same patient in accordance with the single-step RNA isolation method described in 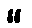 Current Protocols in Molecular Biology 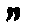 , Chapter 4. The reagent, operating environment, etc. followed the conditions described there.
(2) Total RNA's taken from the hepatic cancer and the non-cancerous portion were each analyzed in an amount of 10 µg each by Northern blot hybridization in accordance with the method using formaldehyde denatured gel electrophoresis described in 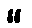 Current Protocols in Molecular Biology 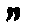 (1987, Greene Publishing Associates and Wiley-Interscience), Chapter 4. The probe DNA used was the same as prepared in Example 3. In autoradiography, the film was Kodak's XAR film, the screen used Du Pont's Lightening Plus (+), and exposure was performed for 24 hours at -80°C.

The results are shown in Fig. 4. In human hepatic cancer, the cancerous area showed a significant increase in the amount of expression compared with the non-cancerous area. This finding demonstrates that the use of the CRTI gene permits monitoring of human hepatic cancer.

### [Example 7] Preparation of anti-CRTI antibodies

1. The CRTI protein prepared in Example 5 was inoculated into rabbits for immunization in accordance with the method described in 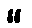 Antibodies, A Laboratory Manual 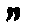 (Cold Spring Harbor Laboratory Press, 1988), Chapter 5, to produce anti-CRTI antibodies.
2. Western blot
   The method described in 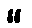 Current protocols in molecular biology 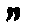 was followed.
   (1) Western blotting of the CRTI protein prepared in Example 5 was performed.
   (2) Then, the anti-CRTI antibodies were reacted with the CRTI protein. The complex was reacted with alkaline phosphatase-labeled anti-rabbit IgG antibodies as secondary antibodies. The alkaline phosphatase was further reacted with a substrate (NBT, BCIP (both products of Promega)) for color development. The anti-CRTI antibodies were confirmed to react with CRTI.

### [Example 8] Investigation of use of CRTI in detecting (diagnosing) hepatic cancer

(1) One gram each of the various cancer tissues prepared in Example 1 was homogenized in 5 ml of 2 x SDS sample buffer, boiled for 3 minutes, and sonicated to obtain the hepatic cancer extract.
(2) The extract was subjected to 12.5% SDS-PAGE electrophoresis, and then Western blotted. The amount of protein in each sample was unified by staining the gels, separately electrophoresed similarly, with Coomassie brilliant blue and comparing the colors.
(3) Then, the blotted extract was reacted with the anti-CRTI antibodies prepared in Example 7. The complex was reacted with alkaline phosphatase-labeled anti-rabbit IgG antibodies as secondary antibodies. The alkaline phosphatase was further reacted with a substrate (NBT, BCID (both products of Promega)) for color development (Fig. 5). As with the results of Northern blot hybridization, significant increases in CRTI protein were confirmed in hepatic cancer and in the process of hepatic cancer occurrence. That is, the antibodies were able to distinguish between hepatic cancer and normal liver, and also distinguish between a liver in the process of carcinogenesis and a normal liver. Thus, they were confirmed to be usable in early diagnosis, etc. of hepatic cancer.

### [Example 9] Investigation of use of CRTI in detecting (diagnosing) cancer in human

(1) The following 9 kinds of cancer cell strains in human were cultured in the media described below:
   (i) Li21 (human hepatic cancer cell strain)
   (ii) LiHM (human hepatic cancer cell strain)
   (iii) LINM (human hepatic cancer cell strain)
   (iv) RERF (human pulmonary cancer cell strain)
   (v) AZ (human gastric cancer cell strain)
   (vi) Hecl (human uterine cancer cell strain)
   (vii) Alex (human hepatic cancer cell strain)
   (viii) MEWO (human melanoma)
   (vix) PaCa (human pancreatic cancer cell strain)

   The cancer cell strains (iv), (v), (vi), (viii) and (vix) were procured from JCRB (Japanese Cancer Research Resource Bank). Instead of these strains (i) to (vix), other strains may be used.
   For the strains (i) to (iii) and (vii), a mixture of RPMI 1640 and 10% FCS was used as a culture medium. The strains (iv) to (vi), (viii) and (vix) were cultured in a medium recommended at the time of procurement.
(2) The cultured cells of each strain incubated in (1) and grown to about 80% of the area of the Petri dish were washed twice with 5 ml of 1 x PBS. Then, 1 ml of 2 x SDS sample buffer was added to each Petri dish measuring 10 cm, and the mixture was homogenized under agitation with Rubber Policeman (Sumitomo Bakelite). The homogenate was transferred to Eppendorf tube, boiled for 5 minutes, and centrifuged to precipitate the insolubles. The supernatant was subjected as a sample to Western blot analysis in the following manner:
   (i) The prepared sample in an amount of 5 to 15 µl was subjected to 10% SDS-PAGE electrophoresis. The amount of the protein in each sample was made equivalent by staining the similarly electrophoresed gel with Coomassie brilliant blue and make quantitative measurement by colorimetry.
   (ii) Then, the extract was reacted with the anti-CRTI antibodies prepared in Example 7. The complex was reacted with alkaline phosphatase-labeled anti-rabbit IgG antibodies as secondary antibodies. The alkaline phosphatase was further reacted with a substrate (NBT, BCIP (both products of Promega)) for color development (Fig. 6). As a result, the expression of CRTI protein was confirmed in all processes of carcinogenesis, including hepatic cancer. That is, the antibodies were confirmed to be capable of detecting CRTI in human cancer cells, and to be usable in diagnosis of cancer, and so forth.

   In the light of the results of Example 8 as well, the antibodies were found to cause cross reaction between humans and rats.

### [Example 10] Confirmation of CRTI gene distribution in rat tissues

To confirm the distribution of the gene in various tissues, Rat MTN Blot™ (CLONTECH) was used. This product is a commercially available membrane blotted with polyA RNA's of rat tissues to be used in the above-mentioned Northern blot hybridization. This membrane was subjected to Northern blot hybridization by the method described in 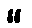 Molecular Cloning Second Edition 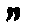 , (Cold Spring Harbor Laboratory Press, 1989) pp. 7.3-7.84 in accordance with the product's instruction manual with the use of the same CRTI gene probe as prepared in Example 3. In autoradiography, the film was Kodak's XAR film, the screen used Du Pont's Lightening Plus(+), and exposure was performed for 24 hours at -80°C.

The results are shown in Fig. 7, which indicates that CRTI gene was expressed in all organs examined, and intensely expressed in the liver and the lung, in particular.

These results and the results of Example 3 suggest that if any organs become cancerous, the expression of CRTI gene may increase. Thus, CRTI gene can be expect to monitor the outbreak and progress of cancer in various organs.

Furthermore, the detection of CRTI protein in various organs permits monitoring of the outbreak and progress of cancer in those organs.

### [Example 11] Confirmation of CRTI gene distribution in human tissues

To confirm the distribution of the gene in various tissues, Human MTN Blot™ (CLONTECH) was used. This product is a commercially available membrane blotted with polyA RNA's of human tissues to be used in the aforementioned Northern blot hybridization. This membrane was subjected to Northern blot hybridization by the method described in 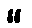 Molecular Cloning Second Edition 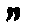 , (Cold Spring Harbor Laboratory Press, 1989) pp. 7.3-7.84 in accordance with the product's instruction manual with the use of the same CRTI gene probe as prepared in Example 3. In autoradiography, the film was Kodak's XAR film, the screen used Du Pont's Lightening Plus(+), and exposure was performed for 24 hours at -80°C.

The results are shown in Fig. 8, which indicates that CRTI gene was expressed in all organs examined, and intensely expressed in the pancreas and liver, in particular.

These results and the results of Example 6 suggest that if any human organs become cancerous, the expression of CRTI gene may increase. Thus, the use of CRTI gene can be expect to monitor the outbreak and progress of cancer in various organs in human.

Furthermore, the detection of CRTI protein in various organs in human permits monitoring of the outbreak and progress of cancer in those organs.

Based on the above experimental results, the detection of the CRTI or CRTI gene of the present invention in a tissue such as the liver makes it possible to diagnose cancer of the tissue, such as hepatic cancer. The use of a part or the whole of the CRTI gene enables the occurrence of cancer, such as hepatic cancer, to be diagnosed at the gene expression level. The use of the anti-CRTI antibodies also permits the immunohistological diagnosis of the outbreak of cancer such as hepatic cancer. Furthermore, the application of the gene or the antibodies to the treatment of cancer can be expected in the future.

### [Example 12] Determination of amino acid sequences of hepatic cancer-specific proteins HRPI and GADII and genes encoding the proteins

### I. HRPI, GADII

1. Large-scale preparation of gene
The following procedure was performed on each of HRPI gene and GADII gene to prepare the genes on a large scale:
(1) The λ phage recovered from the plaques formed on the NZY agar medium in 11.(4) or (5) of Example 1 was suspended in SM solution.
(2) 50 µl of the suspension in (1) and 20 µl of Y1090r-E. coli were mixed, and allowed to stand for 15 minutes at 37°C.
(3) Then, the mixed solution in (2) was transferred to 10 ml NZY medium containing 100 µg/ml ampicillin, and cultured for 6 hours at 37°C.
(4) The culture was centrifuged for 5 minutes at 8,000 rpm, and the supernatant was recovered.
(5) To the supernatant, 1 ml of 5M NaCl and 1.1 g of polyethylene glycol 6000 were added and dissolved.
(6) The solution was placed on ice for 1 hour, and then centrifuged for 20 minutes at 4°C at 10,000 rpm.
(7) The precipitate was recovered and suspended in 700 µl SM solution.
(8) 500 µl chloroform was added to the suspension, and the mixture was stirred to dissolve the remaining E. coli cells.
(9) The solution was centrifuged for 10 minutes at 5,000 rpm, and the aqueous phase was recovered.
(10) To the aqueous phase, 1 mg/ml RNaseA and 5 mg/ml DNaseI (both Sigma) were added in an amount of 1 µl each. After the mixture was left to stand at 37°C for 1 hour, 600 µl of 20% polyethylene glycol 6000 (0.8 M NaCl) was added. The mixture was allowed to stand for 30 minutes on ice.
(11) The system was centrifuged for 20 minutes at 4°C at 15,000 rpm, and then the precipitate was recovered.
(12) To the precipitate, 500 µl SM solution, 50 µl 5M NaCl and 50 µl 0.5M EDTA were added, and 400 µl phenol was further added. The mixture was stirred to dissolve the phage and liberate cDNA.
(13) The solution was centrifuged for 5 minutes at 15,000 rpm at room temperature, and then the aqueous phase was recovered. To the liquid, 1 ml ethanol was added, and the mixture was centrifuged for 20 minutes at 15,000 rpm. The liquid phase was discarded.
(14) The precipitate was washed with 1 ml of 70% ethanol, and dissolved in 100 µl TE solution (10 mM Tris-Cl pH 8.0, 1 mM EDTA) to obtain DNA solution.

2. Insertion of HRPI gene into vector
HRPI gene was inserted into a vector by the following procedure:
(1) A DNA cleavage system of the following composition was prepared to cut DNA with the restriction enzyme NotI (TAKARA SHUZO):

| | |
|---|---|
| DNA solution (prepared in 1.) | 20 µl |
| 0.1% BSA | 10 µl |
| 0.1% TritonX100 | 10 µl |
| NotI (TAKARA SHUZO) | 2 µl |
| Rnase (Nippon Gene) | 1 µl |
| 10xH buffer (Takara) | 10 µl |
| Sterilized water | 47 µl |
| | Total 100 µl |

Reaction temperature 37°C
Reaction time 4 hours

(2) Then, the system was electrophoresed on 0.7% NuSieve (registered trademark) GTG Agarose (TAKARA SHUZO), and DNA of about 1.6 kbp was cut out. This DNA was recovered by means of GENE CLEAN II (registered trademark, Funakoshi) as in its instruction manual.
(3) pBluescriptII (Stratagene) to hold DNA was cleaved with NotI, and then dephosphorylated.
(i) Cleavage with NotI was carried out using the following system:

| | |
|---|---|
| pBluescriptII (1 µg/µl) | 3 µl |
| 10xH buffer | 2 µl |
| 0.1% BSA | 2 µl |
| 0.1% TritonX100 | 2 µl |
| NotI | 2 µl |
| Sterilized water | 10 µl |
| | Total 20 µl |

Reaction temperature 37°C
Reaction time: Overnight

(ii) Then, 2 µl of 1M Tris pH 8.0 was added, and 1 µl Bacterial Alkaline Phosphatase (TAKARA SHUZO) was added, followed by leaving the mixture to stand for 1 hour at 65°C.
(iii) Then, phenol/CHCl₃ extraction was performed twice in accordance with a customary manner to deactivate the enzymes. After purification by ethanol precipitation, the precipitate was dissolved in TE solution to a concentration of 100 ng/µl.
(4) The DNA obtained in (2) and the pBluescriptII obtained in (3) were reacted using the following system to insert the DNA into the vector:

| | |
|---|---|
| DNA (prepared in (2)) | 5 µl |
| NotI-cleaved pBluescriptII (prepared in (3)) | 1 µl |
| 10-fold ligation buffer (Nippon Gene) | 2 µl |
| T4 ligase (Nippon Gene) | 1 µl |
| Sterilized water | 11 µl |
| | Total 20 µl |

Reaction temperature 16°C
Reaction time 2 hours

3. Insertion of GADII gene into vector
GADII gene was inserted into a vector by the following procedure:
(1) A DNA cleavage system of the following composition was prepared to cut DNA with the restriction enzyme EcoRI (TAKARA SHUZO):

| | |
|---|---|
| DNA solution (prepared in 1.) | 20 µl |
| EcoRI (TAKARA SHUZO) | 2 µl |
| Rnase (Nippon Gene) | 1 µl |
| 10xH buffer (TAKARA SHUZO) | 10 µl |
| Sterilized water | 67 µl |
| | Total 100 µl |

Reaction temperature 37°C
Reaction time 4 hours

(2) Then, the system was electrophoresed on 0.7% NuSieve (registered trademark) GTG Agarose (TAKARA SHUZO), and DNA of about 2.1 kbp was cut out. This DNA was recovered by means of GENE CLEAN II (registered trademark, Funakoshi) as in its instruction manual.
(3) pBluescriptII (Stratagene) to hold DNA was cleaved with EcoRI, and then dephosphorylated.
(i) Cleavage with EcoRI was carried out using the following system:

| | |
|---|---|
| pBluescriptII (1 µg/µl) | 3 µl |
| 10xH buffer | 2 µl |
| EcoRI | 2 µl |
| Sterilized water | 14 µl |
| | Total 20 µl |

Reaction temperature 37°C
Reaction time: Overnight

(ii) Then, 2 µl of 1M Tris pH 8.0 was added, and 1 µl Bacterial Alkaline Phosphatase (TAKARA SHUZO) was added, followed by leaving the mixture to stand for 1 hour at 65°C.
(iii) Then, phenol/CHCl₃ extraction was performed twice in accordance with a customary manner to deactivate the enzymes. After purification by ethanol precipitation, the precipitate was dissolved in TE solution to a concentration of 100 µg/µl.
(4) The DNA obtained in (2) and the pBluescriptII obtained in (3) were reacted using the following system to insert the DNA into the vector:

| | |
|---|---|
| DNA (prepared in (2)) | 5 µl |
| EcoRI-cleaved pBluescriptII (prepared in (3)) | 1 µl |
| 10-fold ligation buffer (Nippon Gene) | 2 µl |
| T4 ligase (Nippon Gene) | 1 µl |
| Sterilized water | 11 µl |
| | Total 20 µl |

Reaction temperature 16°C
Reaction time 2 hours

4. Incorporation of gene into E. coli
In accordance with a customary method, the reaction mixture containing the vector fitted with HRPI gene prepared in 2. and the reaction mixture containing the vector fitted with GADII gene prepared in 3. were each wholly mixed with competent cells of E. coli JM109 (TAKARA SHUZO). The mixture was reacted for 30 minutes on ice, for 45 seconds at 42°C, and for 3 minutes on ice. Then, 900 µl of SOC culture medium was added, and the mixture was allowed to stand for 1 hour at 37°C to incorporate the vector into the E. coli JM109. Then, the E. coli JM109 was recovered.
The recombinant E. coli having HRPI gene introduced therein was deposited at the National Institute of Bioscience and Human Technology (Address: 1-3, Higashi 1-chome, Tsukuba-shi, Ibaraki-ken, Japan), Agency of Industrial Science and Technology, Japan, with the accession number FERM P-15164. Then, the microorganism was transferred to international deposition on September 11, 1996 (Accession No. FERM BP-5663). The recombinant E. coli having GADII gene introduced therein was deposited at the same institute under the accession number FERM P-15165, and was then transferred to international deposition on September 11, 1996 (Accession No. FERM BP-5664).
5. Base sequencing of gene
(1) The E. coli JM109 recovered in 4. was sprinkled on LB agar medium (containing 100 µg/ml ampicillin, 0.1 mM IPTG, 0.004% X-gal), and incubated for 16 hours at 37°C.
(2) Of the colonies formed, white colonies were inoculated into 2 ml LB medium (containing 100 µg/ml ampicillin), and cultured for 16 hours at 37°C.
(3) Then, the culture was centrifuged for 1 minute at 12,000 rpm to harvest the cells, and a plasmid DNA solution was recovered by means of Magic Miniprep (registered trademark, Promega).
(4) The recovered DNA was sequenced with T7 Sequencing Kit (Pharmacia) to determine the complete base sequence. The base sequence of HRPI gene is indicated as Seq. ID No. 4 in the Sequence Listing, while the base sequence of GADII gene is indicated as Seq. ID No. 6 in the Sequence Listing. Homology search of the resulting GADII gene sequence through a database revealed CSAD gene as a partially homologous sequence. CSAD gene is reported in Biochemica et Biophysica Acta, vol. 1262, pp. 79-82 (1995).

6. Determination of amino acid sequence
The amino acid sequences of HRPI and GADII were determined by the base sequences determined in 5. The amino acid sequence of HRPI is indicated as Seq. ID No. 3 in the sequence listing, and the amino acid sequence of GADII as Seq. ID No. 5 in the sequence listing.

### II. Human GADII

1. Isolation of full-length gene
Isolation of the full-length gene was performed in accordance with the method described in 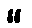 Molecular Cloning Second Edition 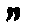 , Chapter 2. Its outline is offered below.
(1) cDNA library used was Human Liver 5'-Stretch plus cDNA library (CLONTECH). The cDNA library was contacted with C600-E. coli, then mixed with NZY medium containing 0.7% agar, and sprinkled on NZY medium plate containing 1.5% agar. Incubation was performed for 6 hours at 42°C to form plaques containing large amounts of cDNA. Then, a nitrocellulose filter (Immobilon (registered trademark, Millipore)) was placed on the plate to transfer the plaques onto the filter.
(2) The filter was treated with sodium hydroxide to denature the cDNA in the plaques. The conditions for denaturation were the same as described in 7.(1) of Example 1.
(3) The denatured cDNA was heat-treated for 2 hours at 75°C to immobilize it, and was used for hybridization. The probe used in the hybridization was the portion from the 65th A to the 611th G of Seq. ID No. 4 in the Sequence Listing of the rat GADII gene whose base sequence had been determined in I.5., that portion being randomly labeled with ³²P-dCTP by means of a random labeling kit of Boehringer Mannheim. The conditions for hybridization and the conditions for washing followed the conditions described in the literature.
(4) As a result of hybridization, full-length human GADII gene was obtained from plaques corresponding to positive signals obtained from cDNA fixed to the filter.

2. Determination of amino acid sequence of human GADII gene and amino acid sequence of human GADII The base sequence of human GADII gene was determined by the same method as in I., i.e., in the following manner:
(1) Large-scale preparation of gene
1) λ phage recovered from the plaques formed on the NZY agar medium in 1.(1) was suspended in SM solution.
2) 50 µl of the suspension in 1) and 20 µl of C600-E. coli were mixed, and allowed to stand for 15 minutes at 37°C.
3) Then, the mixed solution in 2) was transferred to 10 ml NZY medium, and cultured for 6 hours at 37°C.
4) The culture was centrifuged for 5 minutes at 8,000 rpm, and the supernatant was recovered.
5) To the supernatant, 1 ml of 5M NaCl and 1.1 g of polyethylene glycol 6000 were added and dissolved.
6) The solution was placed on ice for 1 hour, and then centrifuged for 20 minutes at 4°C at 10,000 rpm.
7) The precipitate was recovered and suspended in 700 µl SM solution.
8) 500 µl chloroform was added to the suspension, and the mixture was stirred to dissolve the remaining E. coli cells.
9) The solution was centrifuged for 10 minutes at 5,000 rpm, and the aqueous phase was recovered.
10) To the aqueous phase, 1 mg/ml RNaseA and 5 mg/ml DNaseI (both Sigma) were added in an amount of 1 µl each. After the mixture was left to stand at 37°C for 1 hour, 600 µl of 20% polyethylene glycol 6000 (0.8 M NaCl) was added. The mixture was allowed to stand for 30 minutes on ice.
11) The system was centrifuged for 20 minutes at 4°C at 15,000 rpm, and then the precipitate was recovered.
12) To the precipitate, 500 µl SM solution, 50 µl 5M NaCl and 50 µl 0.5M EDTA were added, and 400 µl phenol was further added. The mixture was stirred to dissolve the phage and liberate cDNA.
13) The solution was centrifuged for 5 minutes at 15,000 rpm at room temperature, and then the aqueous phase was recovered. To the liquid, 1 ml ethanol was added, and the mixture was centrifuged for 20 minutes at 15,000 rpm. The liquid phase was discarded.
14) The precipitate was washed with 1 ml of 70% ethanol, and dissolved in 100 µl TE solution (10 mM Tris-Cl pH 8.0, 1 mM EDTA) to obtain DNA solution.

(2) Insertion of human GADII gene into vector
Human GADII gene was inserted into a vector by the following procedure:
1) A DNA cleavage system of the following composition was prepared to cut DNA with the restriction enzyme EcoRI (TAKARA SHUZO):

| | |
|---|---|
| DNA solution (prepared in 1.) | 20 µl |
| EcoRI (TAKARA SHUZO) | 2 µl |
| Rnase (Nippon Gene) | 1 µl |
| 10xH buffer (TAKARA SHUZO) | 10 µl |
| Sterilized water | 67 µl |
| | Total 100 µl |

Reaction temperature 37°C
Reaction time 4 hours

2) Then, the system was electrophoresed on 0.7% NuSieve (registered trademark) GTG Agarose (TAKARA SHUZO), and DNA of about 2.1 kbp was cut out. This DNA was recovered by means of GENE CLEAN II (registered trademark, Funakoshi) as in its instruction manual.
3) pBluescriptII (Stratagene) to hold DNA was cleaved with EcoRI, and then dephosphorylated.
(i) Cleavage with EcoRI was carried out using the following system:

| | |
|---|---|
| pBluescriptII (1 µg/µl) | 3 µl |
| 10xH buffer | 2 µl |
| EcoRI | 2 µl |
| Sterilized water | 14 µl |
| | Total 20 µl |

Reaction temperature 37°C
Reaction time: Overnight

(ii) Then, 2 µl of 1M Tris pH 8.0 was added, and 1 µl Bacterial Alkaline Phosphatase (TAKARA SHUZO) was added, followed by leaving the mixture to stand for 1 hour at 65°C.
(iii) Then, phenol/CHCl₃ extraction was performed twice in accordance with a customary manner to deactivate the enzymes. After purification by ethanol precipitation, the precipitate was dissolved in TE solution to a concentration of 100 µg/µl.
4) The DNA obtained in 2) and the pBluescriptII obtained in 3) were reacted using the following system to insert the DNA into the vector:

| | |
|---|---|
| DNA (prepared in 2)) | 5 µl |
| EcoRI-cleaved pBluescriptII (prepared in 3)) | 1 µl |
| 10-fold ligation buffer (Nippon Gene) | 2 µl |
| T4 ligase (Nippon Gene) | 1 µl |
| Sterilized water | 11 µl |
| | Total 20 µl |

Reaction temperature 16°C
Reaction time 2 hours

(3) Incorporation of gene into E. coli
In accordance with a customary method, the reaction mixture containing the vector fitted with human GADII gene prepared in (2) was wholly mixed with competent cells of E. coli JM109 (TAKARA SHUZO). The mixture was reacted for 30 minutes on ice, for 45 seconds at 42°C, and for 3 minutes on ice. Then, 900 µl of SOC culture medium was added, and the mixture was allowed to stand for 1 hour at 37°C to incorporate the vector into the E. coli JM109. Then, the E. coli JM109 was recovered.
The recombinant E. coli having human GADII gene introduced therein was named hCSAD2, and deposited at the National Institute of Bioscience and Human Technology (Address: 1-3, Higashi 1-chome, Tsukuba-shi, Ibaraki-ken, Japan), Agency of Industrial Science and Technology, Japan, under the accession number FERM P-15762. Then, the microorganism was transferred to international deposition on September 4, 1996 (Accession No. FERM BP-5653).
(4) Base sequencing of gene
1) The E. coli JM109 recovered in (3) was sprinkled on LB agar medium (containing 100 µg/ml ampicillin, 0.1 mM IPTG, 0.004% X-gal), and incubated for 16 hours at 37°C.
2) Of the colonies formed, white colonies were inoculated into 2 ml LB medium (containing 100 µg/ml ampicillin), and cultured for 16 hours at 37°C.
3) Then, the culture was centrifuged for 1 minute at 12,000 rpm to harvest the cells, and a plasmid DNA solution was recovered by means of Magic Miniprep (registered trademark, Promega).
4) The recovered DNA was sequenced with DNA Sequencing Kit (dye terminator method, Perkin-Elmer) to determine the complete base sequence. The base sequence of the DNA is indicated as Seq. ID No. 8 in the Sequence Listing. The homology of the DNA to rat GADII gene (human homology to rat) was 73% for the full length of the gene, and 83% for the protein coding region. The homology was calculated by maximum matching using DINASIS ver 3.0 (Hitachi Software Engineering).
(5) Determination of amino acid sequence
   The amino acid sequence of human GADII was determined by the base sequence determined in (4). This amino acid sequence is indicated as Seq. ID No. 7 in the Sequence Listing. Its homology to rat GADII (homology of human to rat) is 85%.

### [Example 13] Confirmation of possibility for diagnosis of hepatic cancer at gene expression level

1. Analysis of HRPI gene by Northern blot hybridization
   The analysis of HRPI gene by Northern blot hybridization was made in accordance with the method using formaldehyde denatured gel electrophoresis described in 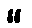 Current Protocols in Molecular Biology 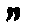 (1987, Greene Publishing Associates and Wiley-Interscience), Chapter 4. PolyA RNA's used in the analysis were (i) polyA RNA derived from a normal liver and that from hepatic cancer 7 months after DEN administration, and (ii) all polyA RNA's of hepatic cancer samples prepared in Example 1. The amount of the polyA RNA used was 500 ng for each sample.
   (1) The HRPI gene carried on pBluescriptII in I.2. of Example 12 was cut out of the vector by treatment with a restriction enzyme.
   (2) Then, the restriction enzyme-treated solution was subjected to agarose gel electrophoresis to separate the targeted HRPI gene.
   (3) The HRPI gene separated in (2) was purified with GENE CLEAN II (registered trademark, Funakoshi).
   (4) The purified HRPI gene was labeled by Random Primed DNA Labeling Kit (Boehringer Mannheim) using α-P³²dCTP (Amersham) in accordance with its instruction manual to make a ³²P-dCTP-labeled HRPI gene probe.
   (5) Systems using the normal liver and the 7-month hepatic cancer were each analyzed by Northern blot hybridization using the probe prepared in (4). As a result, HRPI gene was detected as a gene significantly increased in hepatic cancer. This finding showed that hepatic cancer and normal liver could be distinguished, namely, the occurrence of hepatic cancer could be monitored, and further hepatic cancer could be diagnosed, by using HRPI gene.
   (6) Then, systems using all polyA RNA's of the hepatic cancer samples prepared in Example 1. were each analyzed by Northern blot hybridization using the same probe. In accordance with induction of cancer, significant increases in HRPI gene were noted (Fig. 9). This finding showed that the use of HRPI gene would permit the distinction of hepatic cancer at the initial stage, i.e., the monitoring of progress of hepatic cancer, and further the early diagnosis of hepatic cancer.
2. Analysis of GADII gene by Northern blot hybridization
   GADII gene was also analyzed by Northern blot hybridization in accordance with a similar method. The results are shown in Fig. 10. The results showed that the use of GADII gene would permit distinction between hepatic cancer and normal liver, and the distinction of hepatic cancer at the initial stage, i.e., the monitoring of progress of hepatic cancer, and further the early diagnosis of hepatic cancer.

### [Example 14] Expression of HRPI partial protein

1. Construction of recombinant E. coli
   A partial length clone (the portion encoding from the 185th to 299th amino acids) of the HRPI gene obtained in Example 1.8. was integrated into pET3a vector having a histidine tag incorporated therein (Fig. 3). Then, the vector was introduced into E. coli BL21 (DE3) pLysS.
2. Preparation of HRPI
   (1) The E. coli produced in 1. was cultured in LB medium containing 100 µg/ml ampicillin. When turbidity measured with a spectrophotometer (Beckman) reached 0.5 at a wavelength of 600 nm, IPTG was added in a concentration of 0.5 mM to induce the expression of HRPI protein.
   (2) Two hours later, E. coli cells were recovered, and suspended in Lysis Buffer. The suspension was sonicated, and centrifuged for 15 minutes at 18,000 rpm at 4°C with 50.2 Ti rotor using Beckman Optima XL-80.
   (3) Then, the precipitate was dissolved in 6M guanidine hydrochloride, and purified with Ni-agarose (Qiagen) in accordance with its instruction manual.
3. SDS-PAGE electrophoresis
   The purified sample was subjected to SDS-polyacrylamide electrophoresis, and stained with Coomassie brilliant blue to make sure that purification was successful. The results are shown in Fig. 11.

### [Example 15] Expression of HRPI full-length protein

1. Large-scale preparation of HRPI gene
   (1) Construction of recombinant vector
      HRPI gene was integrated into pBlueBacIII vector (Invitrogen) having a histidine tag incorporated therein as shown in Fig. 12. The details are offered below.
      1) The pBluescriptII vector having HRPI gene inserted therein that was constructed in Example 12.2. was amplified by the PCR method described in 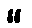 Current Protocols in Molecular Biology 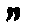 (Greene Publishing Associates and Wiley-Interscience, 1987), Chapter 15. In the amplification, primers of the base sequences of the following formulae (1) and (2) were used, and the base sequence encoding the first methionine portion of HRPI gene was used as NdeI recognition site.
         5' GAA TTC CAT ATG TTC TAT ATA CAG AGT TCT GAG GC 3' ... Formula (1) 5' TCC AGT TAG TGA CGT CTG ATG 3' ... Formula (2)
      2) The amplified gene was cleaved with Ndel and BamHI (both products of TAKARA SHUZO) as under the conditions described in their brochures.
      3) Separately, HRPI gene on the pBluescriptII prepared in Example 12.I.2. was cleaved with BamHI and SacI (both products of TAKARA SHUZO) as under the conditions described in their brochures. Of each of the cleaved genes, only the targeted portion was purified using agarose gel electrophoresis and GENE CLEAN II (registered trademark, Funakoshi).
      4) The genes prepared in 2) and 3) were ligated together by means of Ligation Pack (Nippon Gene) in accordance with its instruction manual.
      5) EcoRI/NdeI/EcoRI adapter (5' AAT TCC ATA TGG 3') was synthesized by a DNA synthesizer, and phosphorylated with T4 polynucleotide kinase (TAKARA SHUZO) in accordance with its instruction manual.
      6) BluescriptII was cleaved with EcoRI, and the EcoRI/Ndel/EcoRI adapter produced in 5) was integrated into the cleavage site.
      7) The Ndel and SacI of the vector constructed in 6) was cleaved, and the gene prepared in 4) was integrated into the cleavage site.
      8) The above vector was introduced into E. coli JM109 in accordance with a customary method, and the E. coli having the vector inserted therein was cultured in LB medium (containing 100 µg/ml ampicillin).
      9) From the cultured E. coli cells, the desired vector was purified by use of Magic Miniprep (registered trademark, Promega) in accordance with its instruction manual.
      10) The vector was cut with NdeI, and then joined to NdeINcoI linker (5' TAT CCA TGG 3') by means of Ligation Pack to turn both cut ends of the vector into NcoI.
      11) The NcoI sites of the HRPI gene portion of the vector were cleaved with a restriction enzyme to prepare HRPI gene with NcoI ends.
      12) After agarose gel electrophoresis of the gene by a customary method, only the desired portion was purified with GENE CLEAN II.
      13) pBlueBacIII vector (Invitrogen) having a histidine tag incorporated therein as shown in Fig. 12 was cut with NcoI by a customary method, and then dephosphorylated.
      14) The HRPI gene purified in 12) and the vector produced in 13) were ligated together by means of Ligation Pack (Nippon Gene) in accordance with its instruction manual.

      Confirmation of the gene integrated into the vector or E. coli in each step described above was performed by reading the base sequence of the insert.
   (2) Large-scale preparation of HRPI gene
      The HRPI gene-integrated plasmid was prepared on a large scale by the method described in 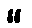 Molecular Cloning Second Edition 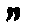 , Cold Spring Harbor Laboratory Press, 1989), Chapter 1. The plasmid was purified by ultracentrifugation described there. This procedure was performed 3 times.
2. Expression of HRPI full-length protein
   (1) The mass produced plasmid was cotransfected into Sf9 cells by lipofection (DO TAP (registered trademark, Boehringer Mannheim)).
   (2) The resulting recombinant virus was again introduced into Sf9 cells to cause large-scale expression. The handling of the Sf9 cells after lipofection, production of the recombinant virus, and large-scale expression of the protein were all performed in compliance with the instruction manual of MAXBAC (registered trademark, Invitrogen).
   (3) The Sf9 cells having expressed HRPI were suspended in SDS sample buffer, boiled for 5 minutes, and then subjected to SDS-PAGE electrophoresis. Then, the electrophoresed sample was stained with Coomassie brilliant blue, whereby a band for HPRI was detected. Fig. 13 shows the results of the SDS-PAGE electrophoresis.

### [Example 16] Expression of GADII

1. Large-scale preparation of GADII gene
   (1) Construction of recombinant vector
      Rat GADII gene was integrated into pET3a vector having a histidine tag incorporated therein as shown in Fig. 3. The details are offered below.
      (i) The pBluescriptII vector having GADII gene inserted therein that was constructed in Example 12.I.3. was amplified by the PCR method described in 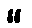 Current Protocols in Molecular Biology 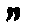 (Greene Publishing Associates and Wiley-Interscience, 1987), Chapter 15. For the amplification, primers of the base sequences of the following formulae (3) and (4) were used, and EcoRI site was introduced ahead of the base sequence encoding the first methionine portion of GADII gene.
         5' GAA TTC CCC ATG GCT GAC TCA AAA CCA CTC AGA A 3' ... Formula (3)
         5' GCA CTG ACC AGA AAT GGC AC 3' ... Formula (4)
      (ii) The amplified gene was cut out by cleavage with EcoRI and SacI.
      (iii) Separately, the remaining portion present on the C-terminal side of GADII gene of GADII gene-inserted pBluescriptII was cut with Bg1II. Then, the residue was smoothed with Klenow fragment, and then cleaved with SacI to cut out the gene.
      (iv) The gene cut out in (ii) and the gene cut out in (iii) were ligated together by means of Ligation Pack (Nippon Gene) in accordance with its instruction manual to produce GADII gene.
      (v) pET3a vector having a histidine tag incorporated therein as shown in Fig. 3 was cut with EcoRI and SmaI, and then dephosphorylated.
      (iv) The GADII gene prepared in (iv) and the vector constructed in (v) were ligated together by means of Ligation Pack (Nippon Gene) in accordance with its instruction manual.

      Confirmation of the gene inserted into the vector during the foregoing process was performed by reading the base sequence of the insert.
   (2) Large-scale preparation of GADII gene
      The GADII gene-integrated plasmid was prepared on a large scale by the method described in 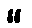 Molecular Cloning Second Edition 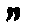 (Cold Spring Harbor Laboratory Press, 1989), Chapter 1.
2. Expression of GADII
   (1) The mass-produced plasmid was introduced into E. coli BL21 (DE3) pLysS.
   (2) The E. coli was cultured in LB medium containing 100 µg/ml ampicillin. When turbidity measured with a spectrophotometer (Beckman) reached 0.5 at a wavelength of 600 nm, IPTG was added in a concentration of 0.5 mM to induce the expression of GADII.
3. Purification of GADII
   (1) Two hours later, E. coli cells were recovered, and suspended in Lysis Buffer. The suspension was sonicated at 4°C, and centrifuged for 15 minutes at 18,000 rpm at 4°C with 50.2 Ti rotor using Beckman Optima XL-80.
   (2) Then, the supernatant was taken, and 1M imidazole (pH 7.5) was added to a final concentration of 10 mM. The mixture was purified with Ni-agarose (registered trademark, Qiagen) under non-denaturing conditions in accordance with its instruction manual.
   (3) The purified sample was subjected to 10% SDS-PAGE electrophoresis, and stained with Coomassie brilliant blue. A band appeared around 56 kD (Fig. 14), confirming the resulting protein to be GADII protein.
   (4) The remaining purified GADII protein was treated with 2xSDS sample buffer and subjected to 5 mm gel thick 10% SDS-PAGE electrophoresis in the same way as in (3). After electrophoresis, the gel was stained with 0.25M KCl for 30 min at 4°C.
   (5) The white-stained target band of about 56 kD was cut out with a cutter knife, and the band was further cut to smaller pieces with the cutter knife.
   (6) The pieces were subjected to Model 422 electroeluator (Bio-Rad) to elute the protein for 8 hours at 20 mA in accordance with its instruction manual.
   (7) The eluted protein was recovered in accordance with the instruction manual of the electroeluator. The protein solution was stored at - 80°C.

### [Example 17] Preparation of anti-HRPI antibodies

1. Preparation of anti-HRPI antibodies
   The HRPI partial protein prepared in Example 14 was inoculated into rabbits for immunization in accordance with the method described in 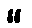 Antibodies, A Laboratory Manual 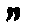 (Cold Spring Harbor Laboratory Press, 1988), Chapter 5, to produce anti-HRPI antibodies.
2. Western blot
   The method described in 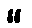 Current protocols in molecular biology 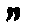 (Greene Publishing Associates and Wiley-Interscience, 1987), Chapter 1 was followed.
   (1) Western blotting of the HRPI partial protein prepared in Example 14 and the HRPI full-length protein prepared in Example 15 was performed.
   (2) Then, the anti-HRPI antibodies were reacted with each of the HRPI partial protein and the HRPI full-length protein. The complexes were each reacted with alkaline phosphatase-labeled anti-rabbit IgG antibodies as secondary antibodies. The alkaline phosphatase was further reacted with a substrate (NBT, BCIP (both products of Promega)) for color development (Fig. 15[A]). The anti-HRPI antibodies were confirmed to react with HRPI. Fig. 15[A] shows that recombinant HRPI has a larger molecular weight by the size of the histidine tag.

### [Example 18] Investigation of use of HRPI in diagnosing hepatic cancer

(1) One gram each of the various cancer tissues prepared in Example 1 was homogenized in 5 ml of 2 x SDS sample buffer, and boiled for 3 minutes to obtain the hepatic cancer tissue extract.
(2) The extract was subjected to 12.5% SDS-PAGE electrophoresis, and then Western blotted. The amounts of protein were unified by staining the gels, separately electrophoresed similarly, with Coomassie brilliant blue and comparing the colors.
(3) Then, the extract was reacted with the anti-HRPI antibodies prepared in Example 17. The complex was reacted with alkaline phosphatase-labeled anti-rabbit IgG antibodies as secondary antibodies. The alkaline phosphatase was further reacted with a substrate (NBT, BCIP (both products of Promega)) for color development (Fig. 15[B]). As with the results of Northern blot hybridization, significant increases in HRPI protein were confirmed in hepatic cancer and in the process of hepatic cancer occurrence. That is, the antibodies were able to distinguish between hepatic cancer and normal liver, and also distinguish between a liver in the process of carcinogenesis and a normal liver. Thus, they were confirmed to be usable in the monitoring of hepatic cancer outbreak and the early diagnosis of hepatic cancer.

### [Example 19] Preparation of anti-GADII antibodies

1. Preparation of anti-GADII antibodies
   The GADII protein prepared in Example 16 was inoculated into rabbits for immunization in accordance with the method described in 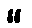 Antibodies, A Laboratory Manual 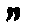 (Cold Spring Harbor Laboratory Press, 1988), Chapter 5, to produce anti-GADII antibodies.
2. Western blot
   The method described in 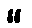 Current protocols in molecular biology 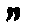 (Greene Publishing Associates and Wiley-Interscience, 1987), Chapter 1 was followed.
   (1) Western blotting of the GADII protein prepared and purified in Example 16 was performed on a nylon membrane (Immobilon P (Millipore)).
   (2) Then, the anti-GADII antibodies were reacted with the GADII protein on the membrane. The complex was reacted with alkaline phosphatase-labeled anti-rabbit IgG antibodies as secondary antibodies. The alkaline phosphatase was further reacted with a substrate (NBT, BCID (both products of Promega)) for color development. A band for GADII protein was detected at 56 kD, confirming the anti-GADII antibodies to react with GADII protein.

### [Example 20] Investigation of use of GADII protein in diagnosing hepatic cancer

(1) One gram each of the hepatic cancer tissue (7 month after DEN administration) and normal liver tissue prepared in Example 1 was homogenized in 5 ml of 2 x SDS sample buffer, and boiled for 3 minutes to obtain the hepatic cancer tissue extract.
(2) The extract and the GADII prepared in Example 16 were subjected to 12.5% SDS-PAGE electrophoresis, and then Western blotted. The amounts of protein were unified by staining the gels, separately electrophoresed similarly, with Coomassie brilliant blue and comparing the colors.
(3) Then, the extract was reacted with the anti-GADII antibodies prepared in Example 19. The complex was reacted with alkaline phosphatase-labeled anti-rabbit IgG antibodies as secondary antibodies. The alkaline phosphatase was further reacted with a substrate (NBT, BCIP (both products of Promega)) for color development. As with the results of Northern blot hybridization, significant increases in GADII protein were confirmed in hepatic cancer and in the process of hepatic cancer occurrence. That is, the antibodies were able to distinguish between hepatic cancer and normal liver, and also distinguish between a liver in the process of carcinogenesis and a normal liver. Thus, they were confirmed to be usable in the monitoring of hepatic cancer outbreak and the early diagnosis of hepatic cancer.

### [Example 21] Confirmation of HRPI gene and GADII gene distribution in tissues

To confirm the distribution of the genes in various tissues, Rat MTN Blot (registered trademark, CLONTECH) was used. This product is a commercially available membrane blotted with polyA RNA's of rat tissues to be used in the above-mentioned Northern blot hybridization. This membrane was subject to Northern blot hybridization by the method described in 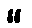 Molecular Cloning Second Edition 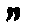 , (Cold Spring Harbor Laboratory Press, 1989) pp. 7.3-7.84 in accordance with the product's instruction manual with the use of the aforementioned HRPI gene probe and GADII gene probe.

The results with the HRPI gene probe are shown in Fig. 16, while the results with the GADII gene probe are shown in Fig. 17. These results indicate that the HRPI gene was intensely expressed in the liver and kidney, and in the lung as well. Whereas the GADII gene was intensely expressed in the liver and kidney, and in the lung as well, although different in length.

Thus, the detection of the HRPI or the HRPI gene or GADII or GADII gene of the present invention in the liver permits the monitoring of the progress of hepatic cancer, and diagnosis of hepatic cancer, especially the early diagnosis of hepatic cancer.

Furthermore, the use of a part or the whole of the HRPI gene or GADII gene makes it possible to diagnose the onset of hepatic cancer at the gene expression level.

Moreover, the use of the anti-HRPI or anti-GADII antibodies permits the immunohistological diagnosis of hepatic cancer occurrence.

In the future, the genes, antisense genes to the genes, or antibodies to the genes can be expected to find use in the treatment of hepatic cancer.

[Sequence Listing]

Seq. ID No.:          1

Length of sequence:   267

Morphology of sequence:      Amino acid

Topology:             Linear

Type of sequence:     Protein

Sequence

Met Val Val Val Ala Ala Ala Pro Ser Ala Ala Ser Ala Ala Pro Lys
 1               5                  10                  15

Val Leu Leu Leu Ser Gly Gln Pro Ala Ser Gly Gly Arg Ala Leu Pro
            20                  25                  30

Leu Met Val Pro Gly Pro Arg Ala Ala Gly Ser Glu Ala Ser Gly Thr
        35                  40                  45

Pro Gln Ala Arg Lys Arg Gln Arg Leu Thr His Leu Ser Pro Glu Glu
    50                  55                  60

Lys Ala Leu Arg Arg Lys Leu Lys Asn Arg Val Ala Ala Gln Thr Ala
65                  70                  75                  80

Arg Asp Arg Lys Lys Ala Arg Met Ser Glu Leu Glu Gln Gln Val Val
                85                  90                  95

Asp Leu Glu Glu Glu Asn Gln Lys Leu Gln Leu Glu Asn Gln Leu Leu
            100                 105                 110

Arg Glu Lys Thr His Gly Leu Val Ile Glu Asn Gln Glu Leu Arg Thr
        115                 120                 125

Arg Leu Gly Met Asn Ala Leu Val Thr Glu Glu Val Ser Glu Ala Glu
    130                 135                 140

Ser Lys Gly Asn Gly Val Arg Leu Val Ala Gly Ser Ala Glu Ser Ala
145                 150                 155                 160
Ala Leu Arg Leu Arg Ala Pro Leu Gln Gln Val Gln Ala Gln Leu Ser
                165                 170                 175
Pro Pro Gln Asn Ile Phe Pro Trp Ile Leu Thr Leu Leu Pro Leu Gln
            180                 185                 190
Ile Leu Ser Leu Ile Ser Phe Trp Ala Phe Trp Thr Ser Trp Thr Leu
        195                 200                 205
Ser Cys Phe Ser Asn Val Leu Pro Gln Ser Leu Leu Ile Trp Arg Asn
    210                 215                 220
Ser Gln Arg Ser Thr Gln Lys Asp Leu Val Pro Tyr Gln Pro Pro Phe
225                 230                 235                 240
Leu Cys Gln Trp Gly Pro His Gln Pro Ser Trp Lys Pro Leu Met Asn
                245                 250                 255
Ser Phe Val Leu Thr Met Tyr Thr Pro Ser Leu
            260                 265

Seq. ID No.:          2
Length of sequence:   2,520
Morphology of sequence:      Nucleic acid
Number of chains:     Double-stranded
Topology:             Linear
Type of sequence:     cDNA to mRNA
Sequence

CTCATTTCCT AAACACTCAT TTTGTGAAAT TCCCCAGGTC TGTGTGTATG TGTTCGCTAA 60
CTCTAAACCG GATATGCCAC CAGTTTGATT TTCGGCTGTA CTAGGGACCG ATGTGGCGCC 120
GGACATTATA ACGTGGTCGG GCTATGGTAA CGATCTGTGA GACTCGGTTT GGAAATCTGG 180
CCTGAGAGGA AAGCCTGGCA TTCCGGGTAA AAGTCTCAGC TGTGTGGGGA CGCGTCTGCC 240
GAGGACCCTG GACCCTGGAC TTCAGCAACC GGCTGTCTCT ACCGTCCACC CACCCTAAGG 300
CCCAGCTCGC ACGGCGAACA GCTGGGCAGC CACGCTGGAC ACTCACCCCG CCCGCGTTGA 360
GCCCGCCCCC GGGCCTGCAG GACCAATAAA CGATGAATAC AGCCGCGCGT CACGCAAGCA 420
[?]AGGCCAATT CGCAGACGGG CACGACCCTA GAAAGGCTGG GCGCGGCAGG CGGCCACGGG 480
GCGGTGGCGG CGCTGGCGTA GACGTTTCCT GGCT 514
ATG GTG GTG GTG GCA GCG GCG CCG AGC GCG GCC TCG GCG GCC CCC AAA 562
GTG CTA CTC CTA TCT GGT CAG CCC GCC TCC GGC GGC CGA GCG CTG CCG 610
CTC ATG GTT CCG GGC CCG CGA GCC GCA GGG TCG GAG GCG AGC GGG ACA 658
CCG CAG GCT CGC AAG CGG CAG CGC CTC ACG CAC CTG AGC CCG GAG GAG 706
AAA GCG CTG CGG AGG AAA CTG AAA AAC AGA GTA GCA GCA CAG ACT GCG 754
CGA GAT AGA AAG AAA GCC CGG ATG AGC GAG CTG GAG CAG CAA GTG GTG 802
GAT TTG GAA GAA GAG AAC CAG AAA CTC CAG CTA GAA AAT CAG CTT TTA 850
CGA GAG AAA ACT CAT GGG CTT GTG ATT GAG AAC CAG GAG TTA AGG ACA 898
CGC TTG GGG ATG AAT GCC CTG GTT ACT GAA GAG GTC TCA GAG GCA GAG 946
TCC AAG GGG AAT GGA GTA AGG CTG GTG GCC GGG TCT GCT GAG TCC GCA 994
GCA CTC AGA CTA CGT GCG CCT CTG CAG CAG GTG CAG GCC CAG TTG TCA 1042
CCT CCC CAG AAC ATC TTC CCA TGG ATT CTG ACG CTG TTG CCT CTT CAG 1090
ATT CTG AGT CTG ATA TCC TTT TGG GCA TTC TGG ACA AGT TGG ACC CTG 1138
TCA TGT TTT TCA AAT GTC CTT CCC CAG AGT CTG CTA ATC TGG AGG AAC 1186
TCC CAG AGG TCT ACC CAG AAG GAC CTA GTT CCT TAC CAG CCT CCC TTT 1234
CTC TGT CAG TGG GGA CCT CAT CAG CCA AGC TGG AAG CCA TTA ATG AAC 1282
TCA TTC GTT TTG ACC ATG TAT ACA CCA AGC CTC TAG 1318

TCTTAGAGAT CCCCTCTGAG ACAGAGAGCC AAACTAATGT GGTAGTGAAA ATTGAGGAAG 1378
CACCTCTAAG CTCTTCAGAA GAGGATCACC CTGAATTCAT TGTCTCAGTG AAGAAAGAAC 1438
CTTTGGATGA TGACTTCATT CCCGAGCTGG GCATCTCAAA CCTGCTTTCA TCCAGCCATT 1498
GTCTGAGACC ACCTTCCTGC CTGCTGGATG CTCACAGTGA CTGTGGATAT GAGGGCTCCC 1558
CTTCTCCCTT CAGCGACATG TCTTCTCCAC TTGGTACAGA CCACTCCTGG GAGGACACTT 1618
TTGCCAACGA ACTTTTCCCC CAGCTGATTA GTGTCTAAAG CCACCCACCA CTGGGCTCCT 1678
TCCCTGATCA TCACACTGCC TAGAGGATAG CATAGGCCTG TCTGCTTCAC TAAAAGCCAA 1738
AGTAGAGGCT ATCTGGCCTT ATAAGAATTC CTCTAAAGTA TTTCAAACCT CTTAGATGAC 1798
TTCCAAGTAT TGTCTTTTGA CACTCAGCTG TCTGAGGTCT TCAAAGGTAT TCCAATACTA 1858
CAGCTTTTGA GATTCTCATT ATCTTAAAGG TGGTAGCATG CTCTAAATCA TAGGGAAAGT 1918
CATCTGACAG TTATCGTTCA GCCTGGCTAT GTAGCCGAGG CTAAGCTGAA ACTTGTGACC 1978
CTCTTGACCC CACTCCCAAG TGCTGGACTT TACCAGGTGT GCAGCTCCAC ACCGGCCTCT 2038
TCACATGTCC TGAAGTAGAC ATGAGAGTCA CCAGTTCTTT CTCTCCTCCC CGCCCCACAG 2098
GTTTCTTTTG TTTCCTTCTA CAAGCAGAGA AACAGCAACC TGAGGGGCCT GTCCTTCCTT 2158
ATGTCCAGTT CAAGTGAAGA TCAAGAATCT TTGTAAAATT ATTGGAAATT TACTGTGTAA 2218
ATGCTTGATG GAATCTTCTT GCTAGTGTAG CTTCTAGAAG GTGCTTTCTC CATTTATTTA 2278
AAACTACCCA TGCAATTAAA AAAGCAACGC AGCATCCCCG TTGAATGATT TTAGGGCTGT 2338
TTATCTTTAT CGTTTGCTAG GGGAGTAATT TCTCATCTAA AGTGAGCACA CCACTTTTTT 2398
AAAAGTCAGA GAGCGGGCTG GGGATTTAGC TCAGTGGTAG AGCGCTTACC TAGGAAGCGC 2458
AAGGCCCTGG GTTCGGTCCC CAGCTCCGGA AAAAAAAGAA CCAAAAAAAA AAAAAAAAAA 2518
AA 2520

Seq. ID No.: 3
Length of sequence: 300
Morphology of sequence: Amino acid
Topology: Linear

Type of sequence: Protein
Sequence
Met Phe Tyr Ile Gln Ser Ser Glu Ala Leu Gln Ile Leu Lys Asn Ser
1               5                   10                  15
Leu Arg Lys His Leu Pro Glu Ser Leu Lys Val Tyr Gly Thr Val Phe
            20                  25                  30
His Met Asn Gln Gly Asn Pro Phe Lys Leu Lys Ala Val Val Asp Lys
        35                  40                  45
Trp Pro Asp Phe Asn Thr Val Val Ile Arg Pro Gln Glu Gln Asp Met
    50                  55                  60
Thr Asp Asp Leu Asp His Tyr Asn Asn Thr Tyr Leu Ile Tyr Ser Lys
65                  70                  75                  80
Asp Pro Lys His Cys Gln Glu Phe Leu Gly Ser Ser Asp Val Thr Asn
                85                  90                  95
Trp Lys Gln His Leu Gln Ile Gln Ser Ser Gln Ser Asp Leu Gly Lys
            100                 105                 110
Val Ile Glu Asn Leu Gly Ala Thr Ser Leu Gly Lys Val Lys His Lys
        115                 120                 125
Gln Cys Phe Leu Tyr Met Val Ser His Thr Ala Lys Lys Leu Thr Pro
    130                 135                 140
Ser Leu Val Asp Ala Lys His Leu Val Val Ser Ser Glu Lys Pro Thr
145                 150                 155                 160
Pro Phe Asp His Gln Leu Phe Lys Phe Ala Arg Leu Asp Val Lys His
                165                 170                 175
Ala Ala Leu Val Asn Ser Ile Trp Tyr Phe Gly Gly Asn Glu Lys Ser
            180                 185                 190

Gln Lys Phe Ile Glu Arg Cys Ile Phe Thr Ser Pro Ser Val Cys Ile
        195                 200                 205
Met Gly Pro Glu Gly Thr Pro Val Ser Trp Ala Leu Met Asp His Thr
    210                 215                 220
Gly Glu Leu Arg Met Ala Gly Thr Leu Pro Lys Tyr Arg His Gln Asn
225                 230                 235                 240
Leu Ile Tyr His Val Ala Phe His Gln Val His Thr Leu Glu Lys Leu
                245                 250                 255
Gly Phe Pro Met Tyr Leu His Val Asp Lys Val Asn Leu Thr Ile Gln
            260                 265                 270
Arg Met Ser Ala Val Leu Gly Met Ser Pro Cys Pro Val Pro Gly Thr
        275                 280                 285
Ser Gly Thr Gly Tyr Leu Ser Lys Ala Arg Lys Glu
    290                 295                 300

Seq. ID No.: 4

Length of sequence: 1634

Morphology of sequence: Nucleic acid

Number of chains: Double-stranded

Topology: Linear

Type of sequence: cDNA to mRNA

[?]equence

ACAGATTTAT AGGTATTAGA AGAG ATG TTC TAT ATA CAG AGT TCT GAG GCA CTG 54

CAG ATT CTG AAG AAT TCC CTA AGG AAG CAC CTC CCT GAG TCC TTA AAG 102

GTT TAT GGG ACT GTC TTC CAC ATG AAC CAG GGA AAC CCA TTC AAG CTC 150

AAG GCT GTG GTG GAC AAG TGG CCT GAT TTT AAT ACT GTT GTT ATT CGA 198

CCA CAG GAA CAG GAC ATG ACA GAT GAC CTT GAC CAC TAC AAC AAC ACT 246

TAC CTA ATT TAT TCC AAG GAT CCC AAG CAC TGT CAG GAA TTC CTT GGC 294

TCA TCA GAC GTC ACT AAC TGG AAA CAA CAT TTG CAG ATC CAA AGT TCA 342

CAG TCA GAC CTG GGC AAA GTG ATA GAA AAC CTT GGA GCC ACT AGC TTG 390

[?]GC AAG GTC AAG CAT AAG CAG TGC TTT CTC TAT ATG GTA TCT CAT ACA 438

GCA AAG AAA CTG ACT CCT TCC TTG GTG GAT GCA AAG CAC TTA GTA GTC 486

AGC AGT GAG AAA CCC ACA CCT TTT GAC CAT CAG TTA TTC AAA TTT GCC 534

CGC CTG GAT GTT AAG CAT GCT GCA TTG GTG AAT AGC ATC TGG TAT TTT 582

GGT GGC AAT GAG AAA AGC CAG AAG TTC ATT GAA CGC TGT ATC TTT ACC 630

TCT CCC AGC GTC TGT ATT ATG GGC CCT GAG GGG ACC CCT GTG TCC TGG 678

GCC CTG ATG GAT CAC ACT GGA GAA CTA AGA ATG GCA GGC ACC TTG CCT 726

AAG TAC CGG CAC CAG AAC CTC ATT TAC CAT GTT GCT TTT CAC CAG GTC 774

CAT ACA CTA GAG AAG CTG GGC TTT CCC ATG TAT TTA CAT GTG GAT AAG 822

GTT AAC CTC ACC ATT CAG AGA ATG TCT GCT GTG CTG GGC ATG TCC CCA 870

TGC CCT GTA CCT GGA ACC AGT GGA ACT GGG TAC CTC TCT AAA GCC AGA 918

AAA GAG TGA GAGGGGGACT GCACTTGTAG ATGGAGAAAT GGCTGAGTGA ACAGAGAGAA 977

GTGAACTGTC ATTAGGGTGG TGTGTGTTTC TAGAGAGCAG GGTTGCATCA CACTGAGCTG 1037

CACACAGTTT CTTCGCCCTC ACATTGCTCA TGCTCTTAAT TCCATTTCTC AGCATACAGA 1097

AGGCCACCAT GTCCTGCTCC CACATTCTCA GGATTCCTGT AACACATTTG GCTAAATTGT 1157

ACTCTCTCCT ATTCATTTTG CAGGCTCATT GATCCATGGC TATTAGTCAG TAGGTTTGTT 1217

TTGTGACCTG ACCCCCCACA ACCCTGCACA CAGGCATACT TCCTCTGGCT TTCTGTAGCT 1277

TTGCTTTTAT GGGTTGGAGA AGTTTGTTCT CATGTCACTC ACCAGACATT AAATTGTTAA 1337

TATGTAAAAA CTACTATACA TGCAAACACA GATACACAGT ATGTGTGGTA CAACTGTAAG 1397

AATTGTTAGA AAAGAGACTA GAGCTCAGCA AGTAAATGTG CCTGCAGTCA AGCCTGGTAA 1457

CCTGAATTTG ATTCCTGGGA ACCACATTGT AAAAGGAGAG AATCTATTCC ATCGACTTGT 1517

CCTCTAACCT CCCCAGGTAT GGGGCGCATG TGCACACACA AATAAGTAAA TGTAATCTTT 1577

AAACAGTTAA AAAGACTAAC TGGTGATCTT TGAGACTAGA TTTGTATTTT TTTTTTC 1634

Seq. ID No.: 5
Length of sequence: 506
Morphology of sequence: Amino acid
Topology: Linear
Type of sequence: Protein
Sequence
Met Ala Asp Ser Lys Pro Leu Arg Thr Leu Asp Gly Asp Pro Val Pro
1               5                   10                  15
Val Glu Ala Leu Leu Arg Asp Val Phe Gly Ile Val Val Asp Glu Ala
            20                  25                  30
Ile Arg Lys Gly Thr Asn Ala Ser Glu Lys Val Cys Glu Trp Lys Glu
        35                  40                  45
Pro Glu Glu Leu Lys Gln Leu Leu Asp Leu Glu Leu Gln Ser Gln Gly
    50                  55                  60
Glu Ser Arg Glu Arg Ile Leu Glu Arg Cys Arg Ala Val Ile His Tyr
65                  70                  75                  80
Ser Val Lys Thr Gly His Pro Arg Phe Phe Asn Gln Leu Phe Ser Gly
                85                  90                  95
Leu Asp Pro His Ala Leu Ala Gly Arg Ile Ile Thr Glu Ser Leu Asn
            100                 105                 110
Thr Ser Gln Tyr Thr Tyr Glu Ile Ala Pro Val Phe Val Leu Met Glu
        115                 120                 125
Glu Glu Val Leu Lys Lys Leu Arg Ala Leu Val Gly Trp Asn Thr Gly
    130                 135                 140
Asp Gly Val Phe Cys Pro Gly Gly Ser Ile Ser Asn Met Tyr Ala Ile
145                 150                 155                 160

Asn Leu Ala Arg Phe Gln Arg Tyr Pro Asp Cys Lys Gln Arg Gly Leu
                165                 170                 175
Arg Ala Leu Pro Pro Leu Ala Leu Phe Thr Ser Lys Glu Cys His Tyr
            180                 185                 190
Ser Ile Thr Lys Gly Ala Ala Phe Leu Gly Leu Gly Thr Asp Ser Val
        195                 200                 205
Arg Val Val Lys Ala Asp Glu Arg Gly Lys Met Ile Pro Glu Asp Leu
    210                 215                 220
Glu Arg Gln Ile Ser Leu Ala Glu Ala Glu Gly Ser Val Pro Phe Leu
225                 230                 235                 240
Val Ser Ala Thr Ser Gly Thr Thr Val Leu Gly Ala Phe Asp Pro Leu
                245                 250                 255
Asp Ala Ile Ala Asp Val Cys Gln Arg His Gly Leu Trp Leu His Val
            260                 265                 270
Asp Ala Ala Trp Gly Gly Ser Val Leu Leu Ser Arg Thr His Arg His
        275                 280                 285
Leu Leu Asp Gly Ile Gln Arg Ala Asp Ser Val Ala Trp Asn Pro His
    290                 295                 300
Lys Leu Leu Ala Ala Gly Leu Gln Cys Ser Ala Leu Leu Leu Arg Asp
305                 310                 315                 320
Thr Ser Asn Leu Leu Lys Arg Cys His Gly Ser Gln Ala Ser Tyr Leu
                325                 330                 335
Phe Gln Gln Asp Lys Phe Tyr Asn Val Ala Leu Asp Thr Gly Asp Lys
            340                 345                 350
Val Val Gln Cys Gly Arg Arg Val Asp Cys Leu Lys Leu Trp Leu Met
        355                 360                 365

Trp Lys Ala Gln Gly Gly Gln Gly Leu Glu Trp Arg Ile Asp Gln Ala
    370                 375                 380
Phe Ala Leu Thr Arg Tyr Leu Val Glu Glu Ile Lys Lys Arg Glu Gly
385                 390                 395                 400
Phe Glu Leu Val Met Glu Pro Glu Phe Val Asn Val Cys Phe Trp Phe
                405                 410                 415
Val Pro Pro Ser Leu Arg Gly Lys Lys Glu Ser Pro Asp Tyr Ser Gln
            420                 425                 430
Arg Leu Ser Gln Val Ala Pro Val Leu Lys Glu Arg Met Val Lys Lys
        435                 440                 445
Gly Thr Met Met Ile Gly Tyr Gln Pro His Gly Thr Arg Ala Asn Phe
    450                 455                 460
Phe Arg Met Val Val Ala Asn Pro Ile Leu Val Gln Ala Asp Ile Asp
465                 470                 475                 480
Phe Leu Leu Gly Glu Ala Gly Ala Ser Gly Pro Gly Pro Val Ser Cys
                485                 490                 495
Phe Leu Ser Leu Pro His Pro Ser Ser Ala
            500                 505

Seq. ID No.: 6
Length of sequence: 2121
Morphology of sequence: Nucleic acid
Number of chains: Double-stranded
Topology: Linear
Type of sequence: cDNA to mRNA
Sequence

CGCGCTCTGA ACCCGTCGTC TGAACCCTCT CTGAACCTTC CTGAAGCTGG AAGATTTCAC 60
CCTG ATG GCT GAC TCA AAA CCA CTC AGA ACC CTG GAT GGG GAC CCT GTG 109
CCT GTG GAG GCT TTG CTC CGG GAC GTG TTT GGG ATT GTC GTA GAT GAG 157
GCC ATT CGG AAG GGG ACC AAT GCC TCT GAG AAG GTC TGC GAA TGG AAG 205
GAG CCT GAA GAG CTC AAG CAG CTG CTG GAC TTG GAG CTG CAG AGC CAG 253
GGC GAG TCT AGG GAG CGG ATC CTG GAG CGC TGC CGG GCT GTG ATT CAT 301
TAC AGT GTC AAG ACT GGT CAC CCC CGG TTC TTC AAC CAG CTC TTC TCA 349
[?]GA TTA GAT CCC CAT GCT CTG GCC GGG CGC ATC ATT ACG GAG AGC CTC 397
AAT ACC AGC CAG TAC ACA TAT GAG ATT GCC CCC GTG TTT GTG CTC ATG 445
GAA GAG GAG GTG CTG AAG AAA CTC CGT GCC CTT GTG GGC TGG AAC ACT 493
GGG GAT GGG GTC TTC TGT CCT GGT GGT TCC ATC TCT AAC ATG TAC GCC 541
ATA AAC CTG GCC CGC TTT CAG CGC TAC CCA GAC TGC AAG CAG AGG GGC 589
CTC CGG GCC CTG CCA CCC TTG GCC CTC TTC ACT TCA AAG GAG TGC CAC 637
TAC TCC ATC ACC AAG GGA GCT GCT TTT CTG GGA CTT GGC ACC GAC AGT 685
GTC CGA GTG GTC AAG GCT GAT GAG AGA GGG AAG ATG ATC CCT GAG GAT 733
CTG GAG AGG CAG ATC AGT CTG GCA GAG GCT GAG GGC TCG GTG CCA TTT 781
[?]TG GTC AGT GCC ACC TCT GGT ACC ACC GTG CTA GGG GCC TTT GAC CCC 829
CTG GAT GCA ATT GCC GAT GTT TGC CAG CGT CAC GGG CTG TGG TTA CAC 877
GTG GAT GCC GCC TGG GGT GGG AGC GTC CTG CTG TCC CGG ACA CAC AGG 925
CAT CTC CTG GAT GGG ATC CAG AGG GCT GAC TCC GTG GCC TGG AAC CCT 973
CAC AAG CTT CTC GCC GCG GGG CTG CAG TGC TCT GCT CTT CTT CTC CGG 1021
GAC ACC TCG AAC CTG CTC AAG CGC TGC CAC GGG TCC CAG GCC AGC TAC 1069
CTC TTC CAG CAA GAC AAG TTC TAC AAC GTG GCT CTG GAC ACC GGA GAC 1117
AAG GTG GTG CAG TGT GGC CGC CGC GTG GAC TGT CTG AAG CTG TGG CTC 1165
ATG TGG AAG GCG CAG GGT GGG CAA GGG CTG GAG TGG CGC ATC GAC CAG 1213
GCC TTT GCT CTC ACT CGG TAC TTG GTG GAG GAG ATA AAA AAG CGG GAA 1261

GGA TTT GAG TTG GTC ATG GAG CCC GAG TTC GTC AAC GTG TGC TTC TGG 1309
TTT GTG CCT CCC AGC CTG CGG GGG AAG AAG GAG AGC CCA GAT TAC AGC 1357
CAG AGG CTG TCT CAG GTG GCC CCT GTG CTC AAG GAG CGC ATG GTG AAG 1405
AAG GGA ACC ATG ATG ATC GGC TAC CAG CCC CAT GGG ACC CGG GCC AAC 1453
TTC TTC CGA ATG GTG GTG GCC AAC CCC ATA CTG GTC CAG GCC GAT ATA 1501
GAC TTC CTT CTG GGC GAG GCT GGA GCG TCT GGG CCA GGA CCT GTG AGC 1549
TGC TTC CTC TCT CTG CCC CAC CCA AGC TCT GCA TAA GCTCCTG GGTTCCCAAA 1602
AGCGACCTTT CTAGGAAACA GTGGCCTTGA CTGTGTGAGC CCCCACACAC TAACTCTCCT 1662
AGCTAAGTAT TGGCTGCCAG ACGGTGTCTA AGCACACTAC AGTCTGTTCT TACGAAATGT 1722
GCTTCTTTTA AGTCGGTCAT AGTGGTACAC ACCGTTAATA CCAGCACTGG GGAGGCAGAG 1782
GCAGACACAA GCAGATCTCT TGAGTTTGAG GCCAGCCTGG TCTACAGAGC TGGCCTACAC 1842
AGAAAAAAAA CCTGTCTCAA AAAAAAAGAA AGGAAGGAAG AAAGAAAGGA AAAGAAAGAA 1902
ATATTTTTCA TTAAGATTAT GTCTATAAAA AATTGTTATT AATATGAGAG ATATGGTACG 1962
ATGTATTAAG AAAGCTAGAT ATGGGGGTTG GGGATTTAGC TCAGTGGTAG AGCCCTTGCC 2022
TAGGAAGCGC AAGGCCCTGG GTTCGGTCCC CAGCTTCGAA AAAAAGGAAC CACAAAAAAA 2082
ACGGCCCGCT CTAGAACTAG TGGATCCCCC GGCCTGCAG 2121

Seq. ID No.: 7
Length of sequence: 493
Morphology of sequence: Amino acid
Topology: Linear
Type of sequence: Protein

Sequence

Met Ala Asp Ser Glu Ala Leu Pro Ser Leu Ala Gly Asp Pro Val Ala
1               5                   10                  15

Val Glu Ala Leu Leu Arg Ala Val Phe Gly Val Val Val Asp Glu Ala
            20                  25                  30

Ile Gln Lys Gly Thr Ser Val Ser Gln Lys Val Cys Glu Trp Lys Glu
        35                  40                  45

Pro Glu Glu Leu Lys Gln Leu Leu Asp Leu Glu Leu Arg Ser Gln Gly
    50                  55                  60

Glu Ser Gln Lys Gln Ile Leu Glu Arg Cys Arg Ala Val Ile Arg Tyr
65                  70                  75                  80

Ser Val Lys Thr Gly His Pro Arg Phe Phe Asn Gln Leu Phe Ser Gly
                85                  90                  95

Leu Asp Pro His Ala Leu Ala Gly Arg Ile Ile Thr Glu Ser Leu Asn
            100                 105                 110

Thr Ser Gln Tyr Thr Tyr Glu Ile Ala Pro Val Phe Val Leu Met Glu
        115                 120                 125

Glu Glu Val Leu Arg Lys Leu Arg Ala Leu Val Gly Trp Ser Ser Gly
    130                 135                 140

Asp Gly Ile Phe Cys Pro Gly Gly Ser Ile Ser Asn Met Tyr Ala Val
145                 150                 155                 160

Asn Leu Ala Arg Tyr Gln Arg Tyr Pro Asp Cys Lys Gln Arg Gly Leu
                165                 170                 175

Arg Thr Leu Pro Pro Leu Ala Leu Phe Thr Ser Lys Glu Cys His Tyr
            180                 185                 190

Ser Ile Gln Lys Gly Ala Ala Phe Leu Gly Leu Gly Thr Asp Ser Val
        195                 200                 205
Arg Val Val Lys Ala Asp Glu Arg Gly Lys Met Val Pro Glu Asp Leu
    210                 215                 220
Glu Arg Gln Ile Gly Met Ala Glu Ala Glu Gly Ala Val Pro Phe Leu
225                 230                 235                 240
Val Ser Ala Thr Ser Gly Thr Thr Val Leu Gly Ala Phe Asp Pro Leu
                245                 250                 255
Glu Ala Ile Ala Asp Val Cys Gln Arg His Gly Leu Trp Leu His Val
            260                 265                 270
Asp Ala Ala Trp Gly Gly Ser Val Leu Leu Ser Gln Thr His Arg His
        275                 280                 285
Leu Leu Asp Gly Ile Gln Arg Ala Asp Ser Val Ala Trp Asn Pro His
    290                 295                 300
Lys Leu Leu Ala Ala Gly Leu Gln Cys Ser Ala Leu Leu Leu Gln Asp
305                 310                 315                 320
Thr Ser Asn Leu Leu Lys Arg Cys His Gly Ser Gln Ala Ser Tyr Leu
                325                 330                 335
Phe Gln Gln Asp Lys Phe Tyr Asp Val Ala Leu Asp Thr Gly Asp Lys
            340                 345                 350
Val Val Gln Cys Gly Arg Arg Val Asp Cys Leu Lys Leu Trp Leu Met
        355                 360                 365
Trp Lys Ala Gln Gly Asp Gln Gly Leu Glu Arg Arg Ile Asp Gln Ala
    370                 375                 380
Phe Val Leu Ala Arg Tyr Leu Val Glu Glu Met Lys Lys Arg Glu Gly
385                 390                 395                 400

Phe Glu Leu Val Met Glu Pro Glu Phe Val Asn Val Cys Phe Trp Phe
                405                 410                 415
Val Pro Pro Ser Leu Arg Gly Lys Gln Glu Ser Pro Asp Tyr His Glu
            420                 425                 430
Arg Leu Ser Lys Val Ala Pro Val Leu Lys Glu Arg Met Val Lys Glu
        435                 440                 445
Gly Ser Met Met Ile Gly Tyr Gln Pro His Gly Thr Arg Gly Asn Phe
    450                 455                 460
Phe Arg Val Val Val Ala Asn Ser Ala Leu Thr Cys Ala Asp Met Asp
465                 470                 475                 480
Phe Leu Leu Asn Glu Leu Glu Arg Leu Gly Gln Asp Leu
                485                 490

Seq. ID No.: 8

Length of sequence: 1926

Morphology of sequence: Nucleic acid

Number of chains: Double-stranded

Topology: Linear

Type of sequence: cDNA to mRNA

Sequence

[?]GGCGCGCCT GTAATCCCAG CACTCTGGGA GACCGAGATT CTTGGTTGAT GCAAATCAAA 60

TAGAGATCCT G ATG GCT GAC TCA GAA GCA CTC CCC TCC CTT GCT GGG GAC 110

CCA GTG GCT GTG GAA GCC TTG CTC CGG GCC GTG TTT GGG GTT GTT GTG 158

GAT GAG GCC ATT CAG AAA GGA ACC AGT GTC TCC CAG AAG GTC TGT GAG 206

TGG AAG GAG CCT GAG GAG CTG AAG CAG CTG CTG GAT TTG GAG CTG CGG 254

AGC CAG GGC GAG TCA CAG AAG CAG ATC CTG GAG CGG TGT CGG GCT GTG 302

ATT CGC TAC AGT GTC AAG ACT GGT CAC CCT CGG TTC TTC AAC CAG CTC 350

TTC TCT GGG TTG GAT CCC CAT GCT CTG GCC GGG CGC ATT ATC ACT GAG 398

AGC CTC AAC ACC AGC CAG TAC ACA TAT GAA ATC GCC CCC GTG TTT GTG 446

CTC ATG GAA GAG GAG GTG CTG AGG AAA CTG CGG GCC CTG GTG GGC TGG 494

AGC TCT GGG GAC GGA ATC TTC TGC CCT GGT GGC TCC ATC TCC AAC ATG 542

TAT GCT GTA AAT CTG GCC CGC TAT CAG CGC TAC CCG GAT TGC AAG CAG 590

AGG GGC CTC CGC ACA CTG CCG CCC CTG GCC CTA TTC ACA TCG AAG GAG 638

TGT CAC TAC TCC ATC CAG AAG GGA GCT GCG TTT CTG GGA CTT GGC ACC 686

GAC AGT GTC CGA GTG GTC AAG GCT GAT GAG AGA GGG AAA ATG GTC CCC 734

GAG GAT CTG GAG AGG CAG ATT GGT ATG GCC GAG GCT GAG GGT GCT GTG 782

CCG TTC CTG GTC AGT GCC ACC TCT GGC ACC ACT GTG CTA GGG GCC TTT 830

GAC CCC CTG GAG GCA ATT GCT GAT GTG TGC CAG CGT CAT GGG CTA TGG 878

CTG CAT GTG GAT GCT GCC TGG GGT GGG AGC GTC CTG CTG TCA CAG ACA 926

CAC AGG CAT CTC CTG GAT GGG ATC CAG AGG GCT GAC TCT GTG GCC TGG 974
AAT CCC CAC AAG CTC CTC GCA GCA GGC CTG CAA TGC TCT GCA CTT CTT 1022
CTC CAG GAT ACC TCG AAC CTG CTC AAG CGC TGC CAT GGG TCC CAG GCC 1070
AGC TAC CTT TTC CAG CAG GAC AAG TTC TAC GAT GTG GCT CTG GAC ACG 1118
GGA GAC AAG GTG GTG CAG TGT GGC CGC CGT GTG GAC TGT CTG AAG CTG 1166
TGG CTC ATG TGG AAG GCA CAG GGC GAT CAA GGG CTG GAG CGG CGC ATC 1214
GAC CAG GCC TTT GTC CTT GCC CGG TAC CTG GTG GAG GAA ATG AAG AAG 1262
[?]GG GAA GGG TTT GAG CTA GTC ATG GAG CCT GAG TTT GTC AAT GTG TGT 1310
TTC TGG TTC GTA CCC CCC AGC CTG CGA GGG AAG CAG GAG AGT CCA GAT 1358
TAC CAC GAA AGG CTG TCA AAG GTG GCC CCC GTG CTC AAG GAG CGC ATG 1406
GTG AAG GAG GGC TCC ATG ATG ATT GGC TAC CAG CCC CAC GGG ACC CGG 1454
GGC AAC TTC TTC CGT GTG GTT GTG GCC AAC TCT GCA CTG ACC TGT GCT 1502
GAT ATG GAC TTC CTC CTC AAC GAG CTG GAG CGG CTA GGC CAG GAC CTG 1550
TGA GCCTTCTCTG TCTTGCTGCC GGCCTTGATA CCACCCCTCA CCCGCAGAGT 1603
CACTGCATTC CCTCCCAGCC TTTGAGGCCG GGTGCAGTGG CTCACGCCTG TAATCCCAGC 1663
ACTTTGGGAG GCCGAGGCGG GTGGATCACT TGAGGTCAGG AGTTCGAGAC CAGCCTGGCC 1723
AATAAGGTGA AACCCTGTCT CTACTAAAAA TACAAAAATT AGCCGAGCAT GGTGGCCTGT 1783
GCCTGTAAAC CCAGCTACTC AGGAGGTTGG GGCAGAATTG CTTGAACCCA GGGGGCAGAG 1843
GTTGCAGTGA GCCGAGATTG CACCCCTGCA CTCCAGGCTG GGCAACAGTA CGAGACTCTG 1903
TTCCAAAAAA AATAAAAAAG CCG 1926

## Claims

1. A protein represented by an amino acid sequence described as Seq. ID No. 1 in the Sequence Listing.

2. A protein represented by an amino acid sequence described as Seq. ID No. 1 in the Sequence Listing in which one or more amino acids has been or have been added, deleted or replaced, and whose expression in a hepatic tissue is increased in cancer cells compared with normal cells.

3. The protein of claim 2, wherein mRNA encoding the protein is increased in cancer cells compared with normal cells at a level detectable by the subtraction method.

4. DNA containing a base sequence encoding the protein of any one of claims 1 to 3.

5. DNA which contains a base sequence encoding at least part of the protein of any one of claims 1 to 3, and which hybridizes with RNA encoding whole length of the protein.

6. DNA containing a base sequence described as Seq. ID No. 2 in the Sequence Listing.

7. DNA containing a base sequence ranging from the 500th base A to the 2520th base A in the base sequence described as Seq. ID No. 2 in the Sequence Listing.

8. DNA containing a base sequence ranging from the 515th base A to the 1315th base C in the base sequence described as Seq. ID No. 2 in the Sequence Listing.

9. DNA containing a base sequence ranging from the 1st base C to the 499th base T in the base sequence described as Seq. ID No. 2 in the Sequence Listing.

10. DNA which comprises consecutive 12 bases or more in a base sequence encoding the protein of any one of claims 1 to 3, and whose GC content is 30 to 70%.

11. DNA which comprises consecutive 16 bases or more in a base sequence encoding the protein of any one of claims 1 to 3, and whose GC content is 30 to 70%.

12. DNA which comprises consecutive 12 bases or more in the base sequence described as Seq. ID No. 2 in the Sequence Listing, and whose GC content is 30 to 70%.

13. DNA which comprises consecutive 16 bases or more in the base sequence described as Seq. ID No. 2 in the Sequence Listing, and whose GC content is 30 to 70%.

14. The DNA of any one of claims 4 to 13, which has been chemically modified.

15. Antisense DNA to the DNA of any one of claims 4 to 13.

16. RNA containing a base sequence encoding the protein of any one of claims 1 to 3.

17. RNA which comprises consecutive 12 bases or more in a base sequence encoding the protein of any one of claims 1 to 3, and whose GC content is 30 to 70%.

18. RNA which comprises consecutive 16 bases or more in a base sequence encoding the protein of any one of claims 1 to 3, and whose GC content is 30 to 70%.

19. The RNA of any one of claims 16 to 18, which has been chemically modified.

20. Antisense RNA to the RNA of any one of claims 16 to 18.

21. Antibodies specifically reactive with the protein of any one of claims 1 to 3.

22. The antibodies of claim 21 which react with human CRTI and rat CRTI.

23. A method for detecting the protein of any one of claims 1 to 3, said method using the antibodies of claim 21 or 22.

24. A method for detecting cancer, comprising detecting the protein of any one of claims 1 to 3, which is present in a tissue of a mammal, by use of the antibodies of claim 21 or 22.

25. The method for detecting cancer of claim 24, wherein the tissue of the mammal is a hepatic tissue.

26. A method for detecting RNA encoding the protein of any one of claims 1 to 3, said method using the DNA of any one of claims 4 to 14 as a probe.

27. A method for detecting cancer, comprising detecting RNA encoding the protein of any one of claims 1 to 3, which is present in a tissue of a mammal, by use of the DNA of any one of claims 4 to 14 as a probe.

28. The method for detecting cancer of claim 27, wherein the tissue of the mammal is a hepatic tissue.

29. A protein represented by an amino acid sequence described as Seq. ID No. 3 in the Sequence Listing.

30. A protein represented by an amino acid sequence described as Seq. ID No. 3 in the Sequence Listing, wherein one or more amino acids has been or have been added, deleted or replaced, and whose expression in a hepatic tissue is increased in cancer cells compared with normal cells.

31. The protein of claim 30, wherein mRNA encoding the protein is increased in cancer cells compared with normal cells at a level detectable by the subtraction method.

32. DNA containing a base sequence encoding the protein of any one of claims 29 to 31.

33. DNA which contains a base sequence encoding at least part of the protein of any one of claims 29 to 31, and which hybridizes with RNA encoding whole length of the protein.

34. DNA containing a base sequence described as Seq. ID No. 4 in the Sequence Listing.

35. DNA containing a base sequence ranging from the 25th base A to the 924th base G in the base sequence described as Seq. ID No. 4 in the Sequence Listing.

36. DNA which comprises consecutive 12 bases or more in a base sequence encoding the protein of any one of claims 29 to 31, and whose GC content is 30 to 70%.

37. DNA which comprises consecutive 16 bases or more in a base sequence encoding the protein of any one of claims 29 to 31, and whose GC content is 30 to 70%.

38. DNA which comprises consecutive 12 bases or more in the base sequence described as Seq. ID No. 4 in the Sequence Listing, and whose GC content is 30 to 70%.

39. DNA which comprises consecutive 16 bases or more in the base sequence described as Seq. ID No. 4 in the Sequence Listing, and whose GC content is 30 to 70%.

40. The DNA of any one of claims 32 to 39, which has been chemically modified.

41. Antisense DNA to the DNA of any one of claims 32 to 39.

42. RNA containing a base sequence encoding the protein of any one of claims 29 to 31.

43. RNA which comprises consecutive 12 bases or more in a base sequence encoding the protein of any one of claims 29 to 31, and whose GC content is 30 to 70%.

44. RNA which comprises consecutive 16 bases or more in a base sequence encoding the protein of any one of claims 29 to 31, and whose GC content is 30 to 70%.

45. The RNA of any one of claims 42 to 44, which has been chemically modified.

46. Antisense RNA to the RNA of any one of claims 42 to 44.

47. Antibodies specifically reactive with the protein of any one of claims 29 to 31.

48. A method for detecting the protein of any one of claims 29 to 31, said method using the antibodies of claim 47.

49. A method for detecting cancer, comprising detecting the protein of any one of claims 29 to 31, which is present in a tissue of a mammal, by use of the antibodies of claim 47.

50. The method for detecting cancer of claim 49, wherein the tissue of the mammal is a hepatic tissue.

51. A method for detecting RNA encoding the protein of any one of claims 29 to 31, said method using the DNA of any one of claims 32 to 40 as a probe.

52. A method for detecting cancer, comprising detecting RNA encoding the protein of any one of claims 29 to 31, which is present in a tissue of a mammal, by use of the DNA of any one of claims 32 to 40 as a probe.

53. The method for detecting cancer of claim 52, wherein the tissue of the mammal is a hepatic tissue.

54. A protein represented by an amino acid sequence described as Seq. ID No. 5 in the Sequence Listing.

55. A protein represented by an amino acid sequence described as Seq. ID No. 5 in the Sequence Listing, wherein one or more amino acids has been or have been added, deleted or replaced, and whose expression in a hepatic tissue is increased in cancer cells compared with normal cells.

56. The protein of claim 55, wherein mRNA encoding the protein is increased in cancer cells compared with normal cells at a level detectable by the subtraction method.

57. DNA containing a base sequence encoding the protein of any one of claims 54 to 56.

58. DNA which contains a base sequence encoding at least part of the protein of any one of claims 54 to 56, and which hybridizes with RNA encoding whole length of the protein.

59. DNA containing a base sequence described as Seq. ID No. 6 in the Sequence Listing.

60. DNA containing a base sequence ranging from the 65th base A to the 1582nd base A in the base sequence described as Seq. ID No. 6 in the Sequence Listing.

61. DNA which comprises consecutive 12 bases or more in a base sequence encoding the protein of any one of claims 54 to 56, and whose GC content is 30 to 70%.

62. DNA which comprises consecutive 16 bases or more in a base sequence encoding the protein of any one of claims 54 to 56, and whose GC content is 30 to 70%.

63. DNA which comprises consecutive 12 bases or more in the base sequence described as Seq. ID No. 6 in the Sequence Listing, and whose GC content is 30 to 70%.

64. DNA which comprises consecutive 16 bases or more in the base sequence described as Seq. ID No. 6 in the Sequence Listing, and whose GC content is 30 to 70%.

65. The DNA of any one of claims 57 to 64, which has been chemically modified.

66. Antisense DNA to the DNA of any one of claims 57 to 64.

67. RNA containing a base sequence encoding the protein of any one of claims 54 to 56.

68. RNA which comprises consecutive 12 bases or more in a base sequence encoding the protein of any one of claims 54 to 56, and whose GC content is 30 to 70%.

69. RNA which comprises consecutive 16 bases or more in a base sequence encoding the protein of any one of claims 54 to 56, and whose GC content is 30 to 70%.

70. The RNA of any one of claims 67 to 69, which has been chemically modified.

71. Antisense RNA to the RNA of any one of claims 67 to 69.

72. Antibodies specifically reactive with the protein of any one of claims 54 to 56.

73. The antibodies of claim 72, which are reactive with rat GADII.

74. A method for detecting the protein of any one of claims 54 to 56, said method using the antibodies of claim 72 or 73.

75. A method for detecting cancer, comprising detecting the protein of any one of claims 54 to 56, which is present in a tissue of a mammal, by use of the antibodies of claim 72 or 73.

76. The method for detecting cancer of claim 75, wherein the tissue of the mammal is a hepatic tissue.

77. A method for detecting RNA encoding the protein of any one of claims 54 to 56, said method using the DNA of any one of claims 57 to 65 as a probe.

78. A method for detecting cancer, comprising detecting RNA encoding the protein of any one of claims 54 to 56, which is present in a tissue of a mammal, by use of the DNA of any one of claims 57 to 65 as a probe.

79. The method for detecting cancer of claim 78, wherein the tissue of the mammal is a hepatic tissue.

80. A protein represented by an amino acid sequence described as Seq. ID No. 7 in the Sequence Listing.

81. A protein represented by an amino acid sequence described as Seq. ID No. 7 in the Sequence Listing, wherein one or more amino acids has been or have been added, deleted or replaced, and whose expression in a hepatic tissue is increased in cancer cells compared with normal cells.

82. The protein of claim 81, wherein mRNA encoding the protein is increased in cancer cells compared with normal cells at a level detectable by the subtraction method.

83. DNA containing a base sequence encoding the protein of any one of claims 80 to 82.

84. DNA which contains a base sequence encoding at least part of the protein of any one of claims 80 to 82, and which hybridizes with RNA encoding whole length of the protein.

85. DNA containing a base sequence described as Seq. ID No. 8 in the Sequence Listing.

86. DNA containing a base sequence ranging from the 72nd base A to the 1550th base G in the base sequence described as Seq. ID No. 8 in the Sequence Listing.

87. DNA which comprises consecutive 12 bases or more in a base sequence encoding the protein of any one of claims 80 to 82, and whose GC content is 30 to 70%.

88. DNA which comprises consecutive 16 bases or more in a base sequence encoding the protein of any one of claims 80 to 82, and whose GC content is 30 to 70%.

89. DNA which comprises consecutive 12 bases or more in the base sequence described as Seq. ID No. 8 in the Sequence Listing, and whose GC content is 30 to 70%.

90. DNA which comprises consecutive 16 bases or more in the base sequence described as Seq. ID No. 8 in the Sequence Listing, and whose GC content is 30 to 70%.

91. The DNA of any one of claims 83 to 90, which has been chemically modified.

92. Antisense DNA to the DNA of any one of claims 83 to 90.

93. RNA containing a base sequence encoding the protein of any one of claims 80 to 82.

94. RNA which comprises consecutive 12 bases or more in a base sequence encoding the protein of any one of claims 80 to 82, and whose GC content is 30 to 70%.

95. RNA which comprises consecutive 16 bases or more in a base sequence encoding the protein of any one of claims 80 to 82, and whose GC content is 30 to 70%.

96. The RNA of any one of claims 93 to 95, which has been chemically modified.

97. Antisense RNA to the RNA of any one of claims 93 to 95.

98. Antibodies specifically reactive with the protein of any one of claims 80 to 82.

99. The antibodies of claim 98, which are reactive with human GADII.

100. A method for detecting the protein of any one of claims 80 to 82, said method using the antibodies of claim 98 or 99.

101. A method for detecting cancer, comprising detecting the protein of any one of claims 80 to 82, which is present in a tissue of a mammal, by use of the antibodies of claim 98 or 99.

102. The method for detecting cancer of claim 102, wherein the tissue of the mammal is a hepatic tissue.

103. A method for detecting RNA encoding the protein of any one of claims 80 to 82, said method using the DNA of any one of claims 83 to 91 as a probe.

104. A method for detecting cancer, comprising detecting RNA encoding the protein of any one of claims 80 to 82, which is present in a tissue of a mammal, by use of the DNA of any one of claims 83 to 91 as a probe.

105. The method for detecting cancer of claim 104, wherein the tissue of the mammal is a hepatic tissue.
